Europäisches Patentamt

European Patent Office

Office européen des brevets

(19)

(11) **EP 0 949 270 A1**

(12) **EUROPEAN PATENT APPLICATION**

(43) Date of publication:
**13.10.1999 Bulletin 1999/41**

(51) Int. Cl.[6]: **C07K 14/47**, C07K 1/107,
C07K 16/18, A61K 38/17,
G01N 33/564

(21) Application number: **98870078.7**

(22) Date of filing: **09.04.1998**

(84) Designated Contracting States:
**AT BE CH CY DE DK ES FI FR GB GR IE IT LI LU
MC NL PT SE**
Designated Extension States:
**AL LT LV MK RO SI**

(71) Applicant: **INNOGENETICS N.V.**
**9052 Gent (BE)**

(72) Inventor:
**The designation of the inventor has not yet been
filed**

Remarks:
- The applicant has subsequently filed a sequence
  listing and declared, that it includes no new matter.
- Claims 11 to 21 are deemed to be abandoned due
  to non-payment of the claims fees (Rule 31 (2)
  EPC).

(54) **Synthetic peptides containing citrulline recognized by rheumatoid arthritis sera as tools for
diagnosis and treatment**

(57)    The present invention relates to a method of
producing certain peptides containing citrulline residues
that constitute immunogenic determinants of antibodies
present in sera from patients with rheumatoid arthritis
and wherein the presence of at least one citrulline is a
prerequisite for reacting with said antibodies. The inven-
tion also relates to a method of producing said antibod-
ies and the use of said peptides for diagnosis and
treatment of rheumatoid arthritis.

EP 0 949 270 A1

**Description**

The present invention relates to certain peptides containing citrulline and that constitute immunogenic determinants of antibodies present in sera from patients with rheumatoid arthritis and wherein the presence of at least one citrulline is a prerequisite for reacting with said antibodies. The invention also relates to the a method of producing said peptides and the use of said peptides for diagnosis and treatment of rheumatoid arthritis and related diseases. The present invention also relates to new filaggrin alleles.

Rheumatoid arthritis (RA) is a major crippling joint disease which is systemic in nature and of unknown aetiology. It affects 1% of the population, with a male to female ratio of 2:3. In terms of morbidity, the most important feature of RA is joint erosion which leads to pain, deformity and in some cases, severe disability. Life expectations in patients with a severe form of the disease are reduced by up to 10 years. RA has all the features of an autoimmune disease, including the presence of a variety of autoantibodies in patients' sera and the capacity to induce illness by transfer of pathogenic T cells in animal models. The classification of the disease can be challenged on the grounds that borderline forms are very common; furthermore inflammation of the joints is not only restricted to RA, but occurs also in other non-autoimmune diseases such as osteoarthritis, reactive arthritis and gout.

As an early diagnosis allows an adjusted treatment which can highly improve life quality of RA patients, it is of great importance for rheumatologists to have reliable diagnostic criteria to their disposal. The diagnosis of RA is initially based on clinical manifestations. Serological support for such a diagnosis is not very well established and is based mainly on the presence of rheumatoid factors (RF). A positive Waaler-Rose or latex fixation RF test has a predictive value and is related to disease with a more severe outcome. However, a substantial number of RA patients are RF seronegative, while on the other hand, RF is also present in other rheumatic diseases including Sjögren's syndrome and systemic lupus erythematosus, in some chronic bacterial and acute viral infections, in certain parasitic diseases and chronic inflammatory diseases, and has furthermore been demonstrated in control sera from healthy persons (Waller et al., 1964; Chen et al., 1987). This rather low specificity of RF necessitates additional testing for a second RA-specific antibody. The prevalence of previously defined antinuclear antibodies such as SSA, RA33 and RNP in RA is low and their clinical utility for the diagnosis of RA has so far been limited (Hassfeld et al., 1993). In contrast, both the antiperinuclear factor (APF) and antikeratin antibodies (AKA) proved to be helpful in this respect. APF are IgG antibodies in sera from RA patients, provoking a typical immunofluorescence staining pattern on human buccal mucosa cells (Nienhuis and Mandema, 1964). Several studies dealt with the diagnostic usefulness of this test (Westgeest et al., 1987; Janssens et al., 1988; Vivino and Maul, 1989; Youinou et al., 1992; Feltkamp et al., 1993). The high specificity of APF for RA of 80-90% and a good sensitivity of 50-99% make it indeed a suitable diagnostic tool. Its value is especially obvious in about one third of the RA patients who do not possess RF activity (Westgeest et al., 1987; Nesher et al., 1992). A second specificity found in sera of RA patients are the AKA, autoantibodies that were first described by Young et al. (1979) and that give rise to a fluorescent staining pattern with the keratinized layer of rat oesophageal epithelium. Despite the lack of biochemical characterization of their target, the antibodies were infered to be antikeratin antibodies on the disputable ground that cytokeratins constitute the major component of the stratum corneum. These antibodies are present in 36-59% of the RA sera and have been found to be highly specific for RA (Johnson et al., 1981; Miossec et al., 1982; Hajiroussou et al., 1985; Vincent et al., 1989). Like APF, they are predominantly of the IgG class (Johnson et al., 1981; Kataaha et al., 1985; Vincent et al., 1990) and they both can be detected in synovial fluid of patients with RA (Youinou et al., 1985; Kirstein et al., 1989; Vivino and Maul, 1990).

The human epidermal protein filaggrin was recently identified with convincing evidence as one of the major targets for reactivity with AKA and APF autoantibodies (Simon et al., 1993; Sebbag et al., 1995). In the initial study, the neutral/acidic form of human filaggrin was demonstrated to react with 75% of a group of 48 RA sera in Western blot (Simon et al., 1993). More recent data (Vincent et al., 1997) showed a diagnostic sensitivity of more than 50% for anti-filaggrin detection with a corresponding specificity of 95% within a group of 492 sera which included 279 RA sera.

Profilaggrin, the precursor of filaggrin is a histidine-rich, insoluble protein of ±400-kDa. It consists of 10-12 filaggrin repeats of 324 amino acids that are separated by a heptamer linker sequence. The highly phosphorylated polyprotein is stored in the keratohyalin granules of the granular layer of the epidermis Upon terminal differentiation of these cells, profilaggrin is dephosphorylated and proteolytically processed by excision of the linker into functional basic filaggrin molecules of 37-kDa (Resing et al., 1989; 1993; Gan et al., 1990). In the lower cornified cells, these units are involved in the aggregation of keratin intermediate filaments, facilitating formation of intermolecular disulfide bonds and yielding the intracellular fibrous matrix of the cornified cells (Dale et al., 1978; Lynley and Dale, 1983; Harding and Scott, 1983; Mack et al., 1993). After filament aggregation, basic arginine residues are converted to citrulline by a peptidylarginine deiminase, which results in a lower affinity of the molecule for cytokeratins (Harding and Scott, 1983). Finally, filaggrin is completely proteolysed into free amino acids, urocanic acid and carboxylic pyrrolidone acid, which play a role in maintaining an optimal level of moisture and absorbing UV light (Scott et al., 1982).

The tandemly arranged filaggrin units are highly polymorphic: there exists a considerable variation in amino acid sequence across individuals and also between the different filaggrin molecules of one person, where up to 15% of differences have been noted (McKinley-Grant et al., 1989; Gan et al., 1990; Markova et al., 1993). Most variations are attributable to single-base changes, but many also involve changes in charge. The human profilaggrin gene system is even polymorphic in size due to allelic differences between individuals in the number of repeats (10, 11 or 12), so that in one person up to 24 different filaggrin molecules can occur (McKinley-Grant et al., 1989; Presland et al., 1992). The amino acid variations, together with the dephosphorylation events are responsable for the marked heterogeneity observed on two-dimensional gels; the conversion of arginine to citrulline is an additional cause of acidification of the molecules.

As the use of natural filaggrin isolated from human tissues is rather unpractical because of batch variability, the laborious preparative isolation and the restriction on the availability of starting material for purification, synthetic peptides and recombinant protein were tested in order to develop a diagnostic test kit for RA. Recombinant filaggrin was expressed in the eukaryotic COS cell system. However, this approach seemed not succesful as none of the four cloned proteins reacted with APF positive patient sera.

It is an aim of the present invention to provide peptides which have a high reactivity for antibodies present in sera from patients with rheumatoid arthritis.

Another aim of the present invention is to provide methods for obtaining said peptides.

Another aim of the present invention is to provide methods of raising antibodies specifically reactive with said peptides.

Another aim of the present invention is to provide methods of raising anti-idiotype antibodies specifically reactive with the afore mentioned antibodies, thereby mimicking said peptides.

Another aim of the present invention is to provide a pharmaceutical composition comprising these peptides, for therapy or diagnosis.

Another aim of the present invention is to provide a diagnostic kit for rheumatoid arthritis.

Another aim of the present invention is to provide new filaggrin alleles and their corresponding amino acid sequences.

All these aims of the present invention are met by the following embodiments of the present invention.

The immunodominant epitopes of filaggrin were identified, which all contained the unusual amino acid citrulline. Synthetic peptides were generated and proved useful for diagnosis of RA.

According to its main embodiment the present invention relates to peptides containing less than 50 amino acids,
comprising fragments of a filaggrin variant, wherein at least one arginine is substituted by citrulline, and that are able to react with antibodies, wherein the presence of said citrulline is crucial for reaction between said peptide and said antibodies, and wherein said antibodies are present in sera from patients with rheumatoid arthritis.

According to a preferred embodiment the present invention relates to peptides as presented in claim 2.

According to a further embodiment the present invention also relates to a peptide and/or chemical structure comprising any of the above mentioned peptides, fused to a linker molecule. The present invention also relates to peptides comprising and/or consisting of tandem repeats of at least two of any of the above mentioned peptides, or branched peptides that comprises at least one of the above mentioned peptides.

According to a more specific embodiment the present invention also relates to a method for producing any of the above mentioned peptides, by classical chemical synthesis, wherein at least one arginine residue is substituted by citrulline, at certain steps during the chemical synthesis. The present invention also relates to a method for producing any of the above mentioned peptides, wherein the primary amino acid sequence is produced by classical chemical synthesis, and wherein said arginine residue is derivatized towards citrulline after chemical synthesis by incubation with peptidylarginine deiminase. The present invention also relates to a method for producing any of the above mentioned peptides comprising the following steps: (i) transforming an appropriate cellular host with a recombinant vector in which a polynucleic acid is inserted comprising the sequence that codes for said peptide under the control of the appropriate regulatory elements such that said peptide or a protein comprising said peptide is expressed and/or secreted, (ii) culturing said transformed cellular host under conditions allowing expression of said protein or peptide and allowing a partial or optimal derivatization of said arginines present in said peptide, towards citrulline residues, and (iii) harvesting said peptide. The present invention also relates to a method for producing any of the above mentioned peptides comprising the following steps: (i) transforming an appropriate cellular host with a recombinant vector in which a polynucleic acid is inserted comprising the sequence that codes for said peptide under the control of the appropriate regulatory elements, such that said peptide or a protein comprising said peptide is expressed and/or secreted, (ii) culturing said transformed cellular host under conditions allowing expression of said protein or said peptide, (iii) harvesting said protein or said peptide, and (iv) derivatizing arginine residues of said protein or said peptide towards citrulline residues. According to a more specific embodiment the present invention also relates to any of the above mentioned methods, wherein said host cell is a bacterial host or yeast or any other eukaryotic host cell which is preferably transformed with a recombinant baculovirus.

According to a preferred embodiment the present invention also relates to an antibody raised upon immunization with any of the above mentioned peptides, with said antibody being specifically reactive with said peptides, and with said antibody being preferably a monoclonal antibody. The present invention also relates to an anti-idiotype antibody raised upon immunization with any antibody as defined above, with said anti-idiotype antibody being specifically reactive with said antibody, thereby mimicking any of the above mentioned peptides, and with said antibody being preferably a monoclonal antibody.

According to a more specific embodiment the present invention also relates to an immunotoxin molecule comprising and/or consisting of a cell recognition molecule being a peptide as defined above, or an antibody as defined above, covalently bound to a toxin molecule or active fragment thereof.

According to a further embodiment the present invention relates to any of the above mentioned peptides or antibodies or immunotoxine molecules or a composition thereof for use as a medicament. Said use can have the purpose of a medicament for treatment or of a diagnosticum for rheumatoid arthritis. The present invention also relates to a diagnostic kit for use in detecting rheumatoid arthritis, wherein said kit comprises at least one of the above mentioned peptides or antibodies, and with said peptide or antibody being possibly bound to a solid support. More preferably said kit is comprising a range of said peptides or said antibodies, possibly in combination with other epitopes that can characterize auto-immune disease, wherein said peptides and/or epitopes are attached to specific locations on a solid substrate. More preferably said solid support is a membrane strip and said polypeptides are coupled to the membrane in the form of parallel lines. It has to be understood that certain peptides, or antibodies as defined above, alternatively, are not attached to a solid support but are provided in the binding solution to be used as competitors and/or to block other antibodies that are present in sera from patients with autoimmune diseases other than rheumatoid arthritis, thereby decreasing or eliminating possible cross-reaction and/or aspecific binding.

By means of epitope mapping, the immunodominant epitopes of filaggrin as occuring in patients with rheumatoid arthritis were identified (see example 1). These epitopes are further characterized by the presence of citrulline residues which result from derivatization of arginine residues. The presence of said citrulline residues is a prerequisite for recognition by antibodies that are present in sera from rheumatoid arthritis. According to its main embodiment, the present invention relates to those peptide fragments of natural filaggrin variants that react with antibodies characteristically present in sera of patients with rheumatoid arthritis and that are further characterized by a post-translational modification, more preferably a derivatization of arginine towards citrulline. The presence of at least one citrulline residue is a prerequisite for recognition by antibodies that are specifically present in sera of patients with rheumatoid arthritis.

Synthetic peptides were generated wherein arginine residues were substituted by citrulline, thus mimicking the epitopes of natural filaggrin variants. These peptides proved useful for diagnosis of rheumatoid arthritis. According to another embodiment the present invention relates to peptides which immunologically mimic the immunogenic determinants of self proteins recognized by the immune system in patients suffering from rheumatoid arthritis. It is therefore anticipated that the presence of one citrulline can be sufficient for specific recognition by some antibodies present in sera of patients with rheumatoid arthritis.

The term 'peptide' as used throughout the specification and claims refers to a polymer of amino acids and does not refer to a specific length of the product; thus, oligopeptides, polypeptides and proteins are included within the definition of 'peptide'. This term also does not exclude post-expression modifications of the peptide, for example, glycosylations, acetylations, phosphorylations and the like. Included within the definition are, for example, peptides containing one or more analogues of an amino acid (including, for example, unnatural amino acids, PNA, etc.), polypeptides with substituted linkages, as well as other modifications known in the art, both naturally occurring and non-naturally occurring.

Whenever the expression "peptide containing less than 50 amino acids" is used, this should be interpreted in a broad sense, as a means of circumscribing an essentially truncated version of the entire immunoreactive protein that still comprises the highly reactive domain characterized by the presence of citrulline residues. These peptides have a length of preferably 40, 30, 25, 20 or less amino acids.

With 'immunogenic determinant' is meant, those chemical groupings comprising a primary amino acid sequence, and secondary modifications of the amino acid residues in a certain three-dimensional arrangement, that together determine the specific reactivity of the entire antigen for a raised antibody. Such antibody can also recognize different chemical groupings, which are then termed to 'immunologically mimic' the immunogenic determinant.

When secondary modifications of a peptide are said to be 'necessary' or 'crucial', or to 'be a prerequisite' for reacting with an antibody, the absence of said secondary modifications will result in a peptide of which the dissociation constant for interaction with said antibody will be at least two orders of magnitude higher than the dissociation constant for the interaction between said antibody and the peptide wherein the secondary modifications are present, preferably three orders of magnitude higher, and more preferably four orders of magnitude higher.

The term 'crossreaction' also refers to the reaction of one antigen with antibodies developed against another antigen or against antibodies that are found in sera from patients with different diseases.

According to a more specific embodiment the present invention relates to those peptides that contain citrulline residues, wherein the presence of said citrulline residues is crucial for high-affinity interaction with antibodies that are characteristically present in sera of patients with rheumatoid arthritis.

In a more specific embodiment, the present invention relates to a peptide that is characterized by the amino acid sequence

HSASQDGQDTIRGHPGSS or,
HSGIGHGQASSAVRDSGHRGYS or,
DSGHRGYSGSQASDNEGH or,
HSTSQEGQDTIHGHRGS or,
GGQGSRHQQAR or,
QGSRHQQARDSSRHSTSQEGQDTIHGHRGS or,
QGSRHQQARDSSRHSASQDGQDTIRGHPGSS or,
HSGIGHGQASSAVRDSGHRGYSGSQASDNEGH or,
wherein at least one and preferably each arginine is derivatized towards citrulline residues, thereby mimicking the main immunogenic determinant of filaggrin.

The present invention also relates to molecular structures in which at least part represents a peptide or antibody as defined above. Such molecular structures can result from fusion of peptides of the present invention with peptides and/or proteins and/or other molecules that are further characterized in that they -specifically interact with other peptides and/or proteins and/or molecular structures, enabling tagging and/or binding of the fused polypeptide and/or protein to specific tissue- or cell types or that allow for purification of said molecular structures due to the presence of for instance 4, or 5 or 6 consecutive histidine residues, or

- are cytotoxic to T-cells and/or B-cells such as cholera toxin, or
- allow for labelling by means of a radioactive or fluorescent or immunogold or enzymatic marker.

It may also be desirable in certain instances to join two or more peptides together in one peptide structure, or to create branched peptides. One advantage of this arrangement is well known in the art and relates to diagnosis. When antigens are used in an assay in order to detect the antibodies present, tandem repeats or branched peptides of the antigens can increase the amount of immobilized antigens presented to the antibodies and thereby increase the sensitivity of the assay. The sensitivity can be increased exponentially when the immobilized antigens are used together with a specific concentration of such antigens in a soluble form, thereby inducing the formation of crosslinked antigen-immunoprecipitates. A second advantage relates to therapy. The deposition of self-antigen autoimmune complexes in various tissues is an important step towards the acquisition of a pathological condition. It is generally accepted that the main cause of said deposition is the insufficient blood clearance by the liver of the antigen-immune complexes due to the small size of said complexes. Administration of tandem repeats or branched forms of said peptides could increase the size of the formed antigen-immune complexes, and thereby increases the clearance and thus decreases the deposition of said complexes.

The present invention also relates to circularized forms of said peptides, the advantage being well known in the art, and relating to an increased affinity of a conformationally constraint peptide as compared with the more randomly coiled forms of linear peptides.

In order to accommodate for eventual negative characteristics of the claimed peptides, such as rapid degradation, solubility, cytotoxic effects and so on, the skilled person will be able to design conservative as well as non-conservative amino acid substitutions, or substitutions with non-natural amino acids, etc... These will generally account for less than 35 percent of a specific sequence. Such peptides also include peptides with substituted linkages, as well as other modifications known in the art, both naturally occurring and non-naturally occurring. It may be desirable in cases where the filaggrin peptides of the present invention are highly polymorphic, to vary one or more of the amino acids so as to better mimic the different epitopes, or as recognized by antibodies in sera from patients with rheumatoid arthritis.

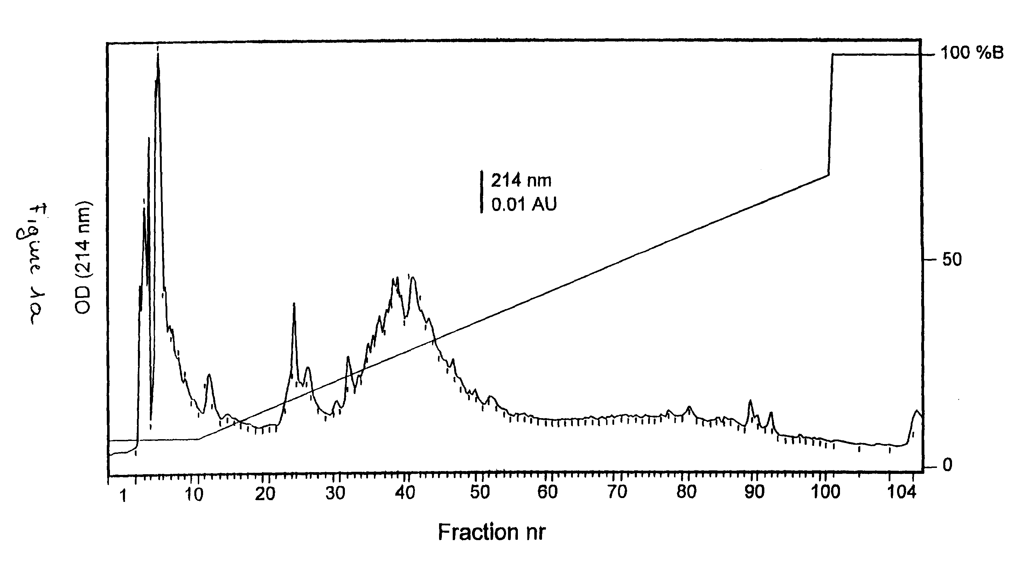

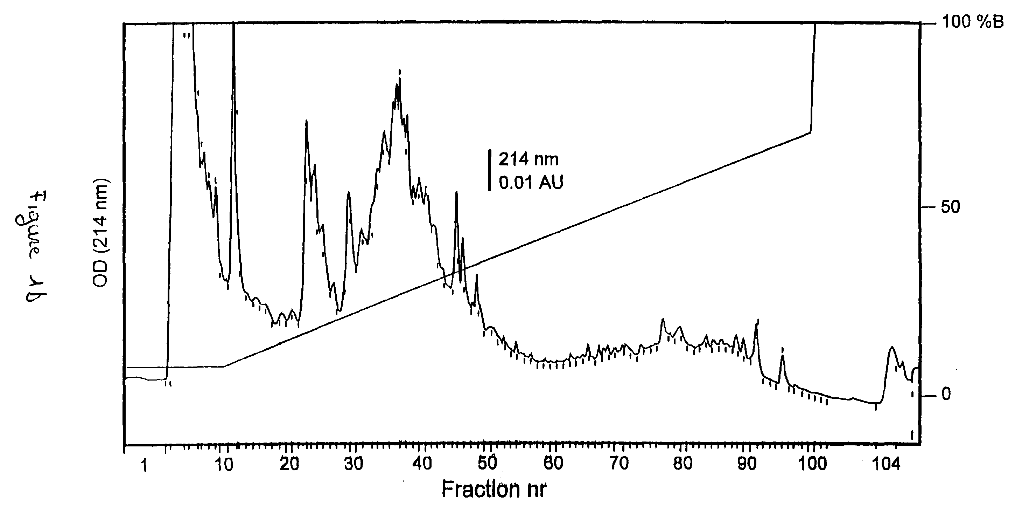

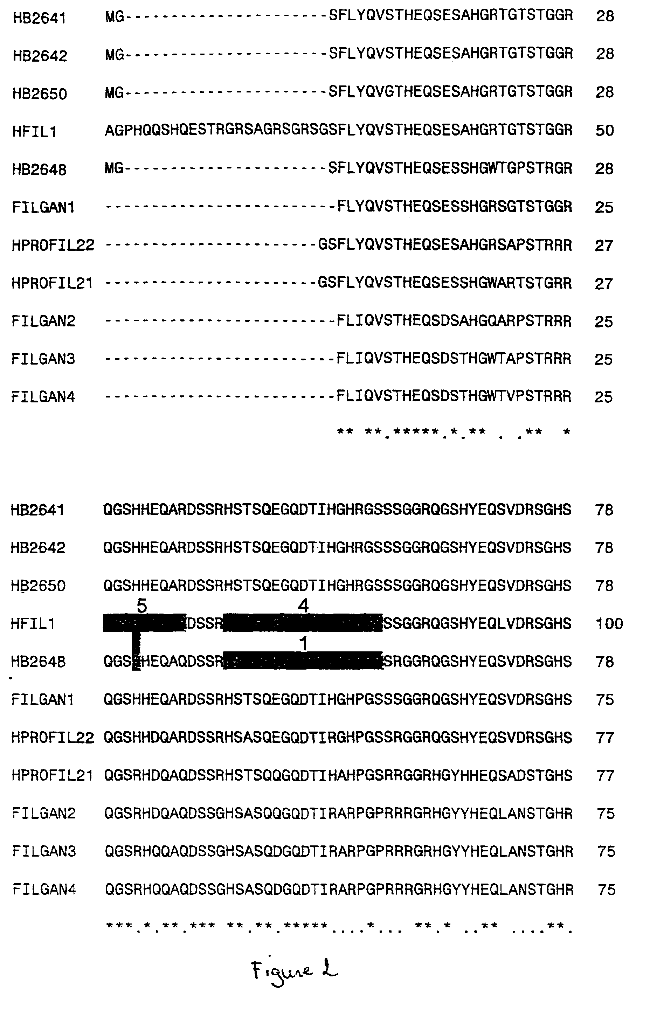

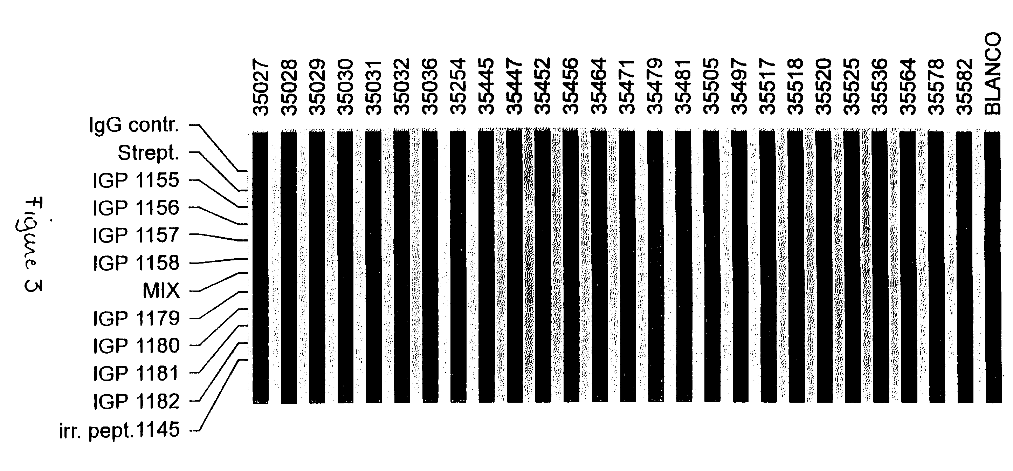

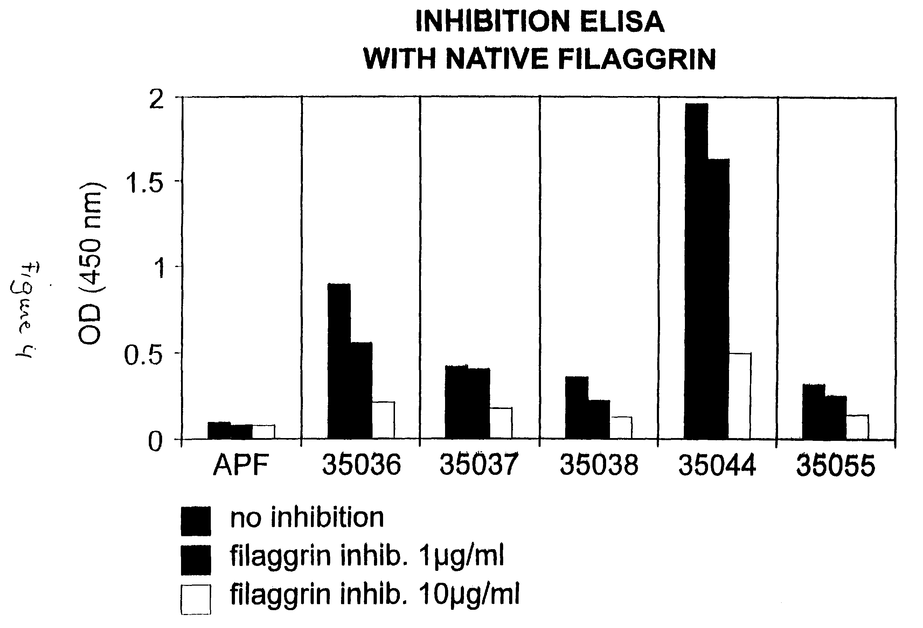

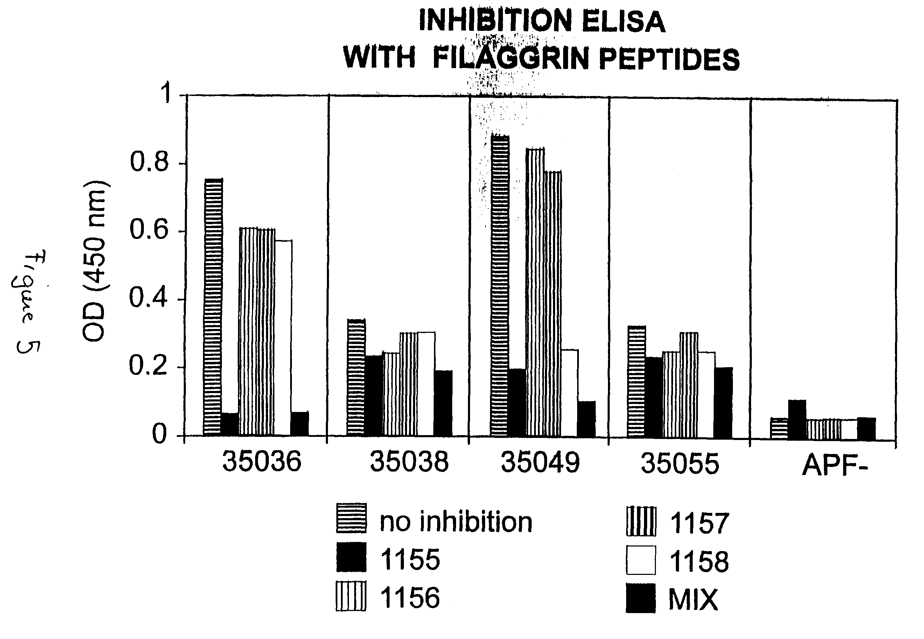

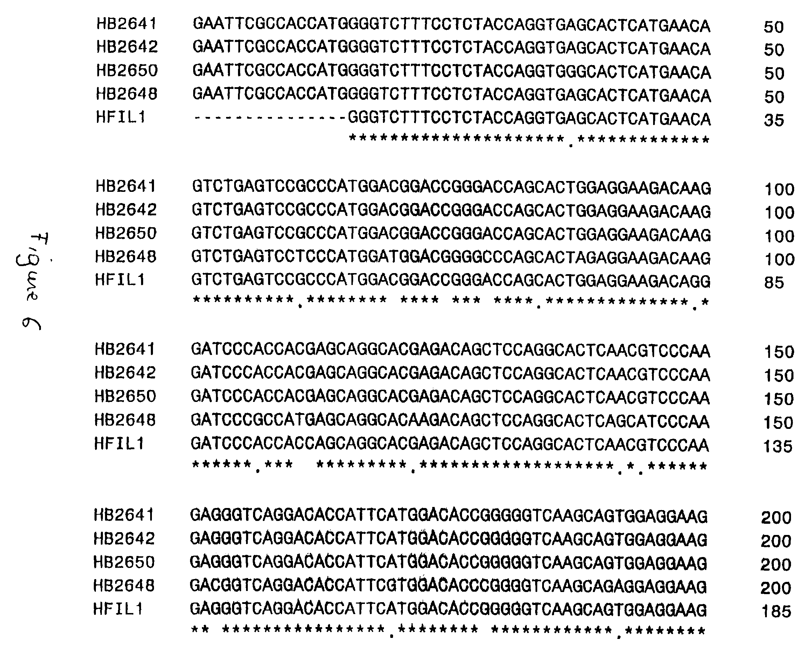

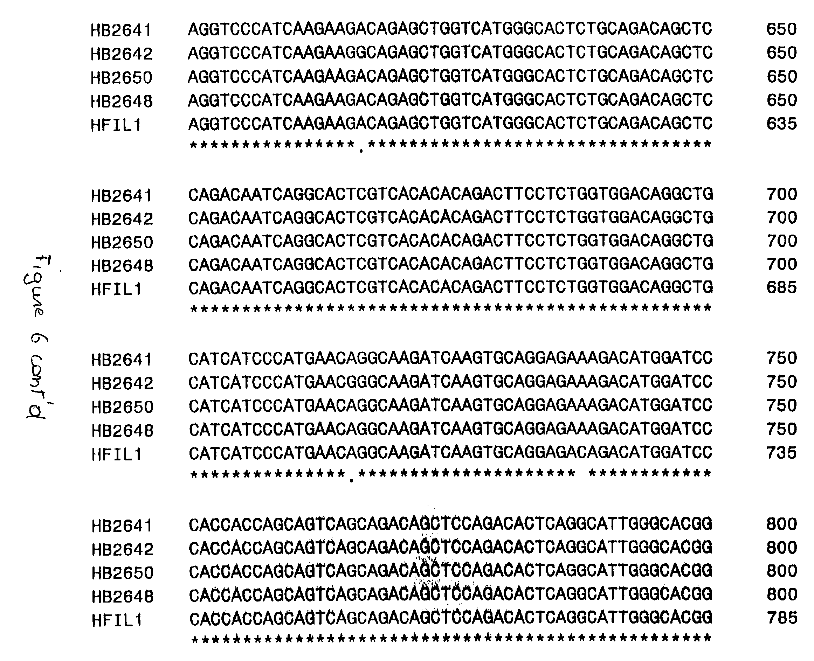

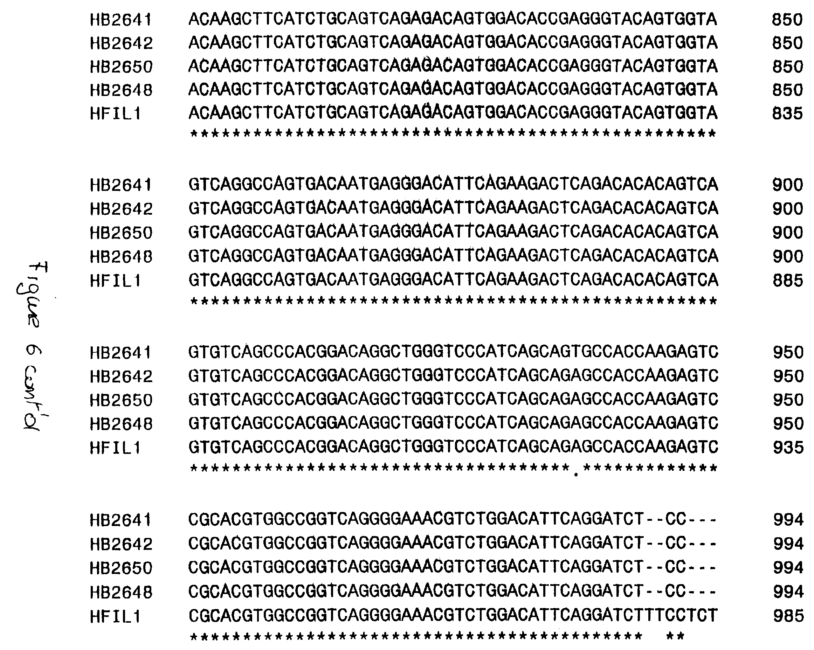

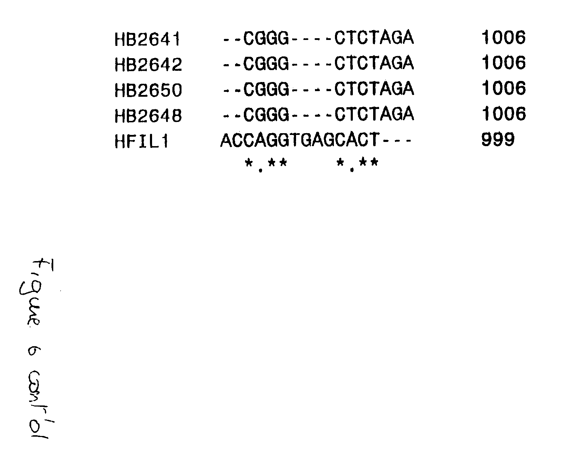

According to another embodiment the present invention also relates to the new allelic variants that were isolated , cloned and sequenced, characterized by the DNA sequence as presented in figure 7 and the amino acid sequence as presented in figure 2. The present invention also relates to any analogs of the peptides of the present invention.

The term "analog" as used throughout the specification or claims to describe the proteins or peptides of the present invention, includes any protein or peptide having an amino acid residue sequence substantially identical to a sequence specifically shown herein in which one or more residues have been conservatively substituted with a biologically equivalent residue. Examples of conservative substitutions include the substitution of hydrophobic residue such as isoleucine, valine, leucine or methionine for another, the substitution of one hydrophilic residue for another such as between arginine and lysine, between glutamine and asparagines, between glycine and serine, the substitution of one basic residue such as lysine, arginine or histidine for another, or the substitution of one acidic residue, such as aspartic acid or glutamic acid for another. Examples of allowable mutations according to the present invention can be found in Table 1.

The phrase "conservative substitution" also includes the use of a chemically derivatized residue in place of a non-derivatized residue provided that the resulting protein or peptide is biologically equivalent to the protein or peptide of the invention.

"Chemical derivative" refers to a protein or peptide having one or more residues chemically derivatized by reaction of a functional side group or peptides with substituted linkages, as well as other modifications known in the art, both naturally occurring and non-naturally occurring. Examples of such derivatized molecules, include but are not limited to, those molecules in which free amino groups have been derivatized to form amine hydrochlorides, p-toluene sulfonyl groups, carbobenzoxy groups, t-butyloxycarbonyl groups, chloracetyl groups or formyl groups. Free carboxyl groups may be derivatized to form salts, methyl and ethyl esters or other types of esters or hydrazides. Free hydroxyl groups may be derivatized to form O-acyl or O-alkyl derivatives. The imidazole nitrogen of histidine may be derivatized to form N-imbenzylhistidine. Also included as chemical derivatives are those proteins or peptides which contain one or more naturally-occurring amino acid derivatives of the twenty standard amino acids. For examples : 4-hydroxyproline may be substituted for proline; 5-hydroxylysine may be substituted for lysine; 3-methylhistidine may be substituted for histidine; homoserine may be substituted for serine; and ornithine may be substituted for lysine. The peptides of the present invention also include any protein or peptide having one or more additions and/or deletions or residues relative to the sequence of a peptide whose sequence is shown herein, as long as the peptide is biologically equivalent to the proteins or peptides of the invention.

| Amino acids | Synonymous groups |
|---|---|
| Ser (S) | Ser, Thr, Gly, Asn |
| Arg (R) | Arg, His, Lys, Glu, Gln |
| Leu (L) | Leu; Ile, Met, Phe, Val, Tyr |
| Pro (P) | Pro, Ala, Thr, Gly |
| Thr (T) | Thr, Pro, Ser, Ala, Gly, His, Gln |
| Ala (A) | Ala, Pro, Gly, Thr |
| Val (V) | Val, Met, Ile, Tyr, Phe, Leu, Val |
| Gly (G) | Gly, Ala, Thr, Pro, Ser |
| Ile (I) | Ile, Met, Leu, Phe, Val, Ile, Tyr |
| Phe (F) | Phe, Met, Tyr, Ile, Leu, Trp, Val |
| Tyr (Y) | Tyr, Phe, Trp, Met, Ile, Val, Leu |
| Cys (C) | Cys, Ser, Thr, Met |
| His (H) | His, Gln, Arg, Lys, Glu, Thr |
| Gln (Q) | Gln, Glu, His, Lys, Asn, Thr, Arg |
| Asn (N) | Asn, Asp, Ser, Gln |
| Lys (K) | Lys, Arg, Glu, Gln, His |

| | |
|---|---|
| Asp (D) | Asp, Asn, Glu, Gln |
| Glu (E) | Glu, Gln, Asp, Lys, Asn, His, Arg |
| Met (M) | Met, Ile, Leu, Phe, Val |

Table 1     Overview of the amino acid substitutions which could form the basis of analogs (muteins) as defined above.

Furthermore, additional amino acids or chemical groups may be added to the amino- or carboxyl terminus for the purpose of creating a "linker arm" by which the peptide can conveniently be attached to a carrier. The linker arm will be at least one amino acid and may be as many as 60 amino acids but will most frequently be 1 to 10 amino acids. The nature of the attachment to a solid phase or carrier can be non-covalent as well as covalent. Possible arrangements of this nature are well described in the art. Natural amino acids such as histidine, cysteine, lysine, tyrosine, glutamic acid, or aspartic acid may be added to either the amino- or carboxyl terminus to provide functional groups for coupling to a solid phase or a carrier. However, other chemical groups such as, for example, biotin and thioglycolic acid, may be added to the termini which will endow the peptides with desired chemical or physical properties. The termini of the peptides may also be modified, for example, by N-terminal acetylation or terminal carboxy-amidation. In each instance, the peptide will preferably be as small as possible while still maintaining substantially all of the sensitivity of the larger peptide.

The peptides of the invention, and particularly the fragments, can be prepared by classical chemical synthesis. The synthesis can be carried out in homogeneous solution or in solid phase. For instance, the synthesis technique in homogeneous solution which can be used is the one described by Houbenweyl in the book entitled "Methode der organischen chemie" (Method of organic chemistry) edited by E. Wunsh, vol. 15-I et II. THIEME, Stuttgart 1974. The polypeptides of the invention can also be prepared in solid phase according to the methods described by Atherton and Shepard in their book entitled "Solid phase peptide synthesis" (IRL Press, Oxford, 1989). The forms of the claimed peptides can be obtained by substituting the citrulline residues for the original arginine derivatives during the classical chemical synthesis, or by contacting the peptides after synthesis with a peptidylarginine deiminase of any eukaryotic origin.

The polypeptides according to this invention can also be prepared by means of recombinant DNA techniques as described by Maniatis et al., Molecular Cloning: A Laboratory Manual, New York, Cold Spring Harbor Laboratory, 1982) by insertion of a polynucleic acid sequence encoding the claimed peptides or part of the claimed peptides in an appropriate vector and transforming a suitable host with said vector. This recombinant expression vector comprises a polynucleic acid or a part thereof as defined above, operably linked to prokaryotic, eukaryotic or viral transcription and translation control elements. In addition this sequence can be operably linked with sequences that allow for secretion of the claimed peptides. The term 'vector' may comprise a plasmid, a cosmid, a phage or a virus or a transgenic organism. Particularly useful may be BCG or adenoviral vectors, as well as avipox recombinant viruses.

The recombinant peptides can be derivatized *in vitro*, by contacting the expressed and/or secreted peptides with a of any eukaryotic origin, or *in vivo* by choosing the appropriate host, like yeast, or any eukaryotic cell, and more preferably by using the baculovirus transformation system, or by coexpressing said peptides with recombinant peptidylarginine deiminase.

Also any of the known purification methods for recombinant peptides can be used for the production of the recombinant peptides of the present invention.

The present invention also relates to a recombinant expression vector comprising a polynucleic acid or a part thereof as defined above, operably linked to prokaryotic, eukaryotic or viral transcription and translation control elements.

In general, said recombinant vector will comprise a vector sequence, an appropriate prokaryotic, eukaryotic or viral promoter sequence followed by a nucleotide sequence encoding a peptide as defined above, with said recombinant vector allowing the expression and/or secretion of any one of the polypeptides as defined above in a prokaryotic, or eukaryotic host or in living mammals when injected as naked DNA.

Also any of the known purification methods for recombinant proteins may be used for the production of the recombinant polypeptides of the present invention.

The term "vector" may comprise a plasmid, a cosmid, a phage, or a virus or a transgenic animal. Particularly useful for vaccine development may be BCG or adenoviral vectors, as well as avipox recombinant viruses.

The present invention also relates to a method for the production of a recombinant polypeptide as defined above, comprising:

- transformation of an appropriate cellular host with a recombinant vector, in which a polynucleic acid or a part thereof according to as defined above has been inserted under the control of appropriate regulatory elements,
- culturing said transformed cellular host under conditions enabling the expression and/or secretion of said insert, and,
- harvesting said polypeptide.

The term "recombinantly expressed" used within the context of the present invention refers to the fact that the proteins of the present invention are produced by recombinant expression methods be it in prokaryotes, or lower or higher eukaryotes as discussed in detail below.

The term "lower eukaryote" refers to host cells such as yeast, fungi and the like. Lower eukaryotes are generally (but not necessarily) unicellular. Preferred lower eukaryotes are yeasts, particularly species within Saccharomyces, Schizosaccharomyces, Kluveromyces, Pichia (e.g. Pichia pastoris), Hansenula (e.g. Hansenula polymorpha), Yarowia, Schwaniomyces, Schizosaccharomyces, Zygosaccharomyces and the like. Saccharomyces cerevisiae, S. carlsbergensis and K. lactis are the most commonly used yeast hosts, and are convenient fungal hosts.

The term "prokaryotes" refers to hosts such as E.coli, Lactobacillus, Lactococcus, Salmonella, Streptococcus, Bacillus subtilis or Streptomyces. Also these hosts are contemplated within the present invention.

The term "higher eukaryote" refers to host cells derived from higher animals, such as mammals, reptiles, insects, and the like. Presently preferred higher eukaryote host cells are derived from Chinese hamster (e.g. CHO), monkey (e.g. COS and Vero cells), baby hamster kidney (BHK), pig kidney (PK15), rabbit kidney 13 cells (RK13), the human osteosarcoma cell line 143 B, the human cell line HeLa and human hepatoma cell lines like Hep G2, and insect cell lines (e.g. Spodoptera frugiperda). The host cells may be provided in suspension or flask cultures, tissue cultures, organ cultures and the like. Alternatively the host cells may also be transgenic animals.

The term "recombinant polynucleotide" or "nucleic acid" intends a polynucleotide or nucleic acid of genomic, cDNA, semisynthetic, or synthetic origin which, by virtue of its origin or manipulation : (1) is not associated with all or a portion of a polynucleotide with which it is associated in nature, (2) is linked to a polynucleotide other than that to which it is linked in nature, or (3) does not occur in nature.

The term "recombinant host cells", "host cells", "cells", "cell lines", "cell cultures", and other such terms denoting microorganisms or higher eukaryotic cell lines cultured as unicellular entities refer to cells which can be or have been, used as recipients for a recombinant vector or other transfer polynucleotide, and include the progeny of the original cell which has been transfected. It is understood that the progeny of a single parental cell may not necessarily be completely identical in morphology or in genomic or total DNA complement as the original parent, due to natural, accidental, or deliberate mutation.

The term "replicon" is any genetic element, e.g., a plasmid, a chromosome, a virus, a cosmid, etc., that behaves as an autonomous unit of polynucleotide replication within a cell; i.e., capable of replication under its own control.

The term "vector" is a replicon further comprising sequences providing replication and/or expression of a desired open reading frame.

The term "control sequence" refers to polynucleotide sequences which are necessary to effect the expression of coding sequences to which they are ligated. The nature of such control sequences differs depending upon the host organism; in prokaryotes, such control sequences generally include promoter, ribosomal binding site, splicing sites and terminators; in eukaryotes, generally, such control sequences include promoters, splicing sites, terminators and, in some instances, enhancers. The term "control sequences" is intended to include, at a minimum, all components whose presence is necessary for expression, and may also include additional components whose presence is advantageous, for example, leader sequences which govern secretion.

The term "promoter" is a nucleotide sequence which is comprised of consensus sequences which allow the binding of RNA polymerase to the DNA template in a manner such that mRNA production initiates at the normal transcription initiation site for the adjacent structural gene.

The expression "operably linked" refers to a juxtaposition wherein the components so described are in a relationship permitting them to function in their intended manner. A control sequence "operably linked" to a coding sequence is ligated in such a way that expression of the coding sequence is achieved under conditions compatible with the control sequences.

The polynucleic acids encoding the peptides of the present invention and inserted into the vector sequence may be attached to a signal sequence. Said signal sequence may be that from any source, e.g. the IgG or tissue plasminogen activator (tpa) leader sequence for expression in mammalian cells, or the $\alpha$-mating factor sequence for expression into yeast cells.

A variety of vectors may be used to obtain the peptides of the present invention. Lower eukaryotes such as yeasts and glycosylation mutant strains are typically transformed with plasmids, or are transformed with a recombinant virus. The vectors may replicate within the host independently, or may integrate into the host cell genome.

Higher eukaryotes may be transformed with vectors, or may be infected with a recombinant virus, for example a recombinant vaccinia virus. Techniques and vectors for the insertion of foreign DNA into vaccinia virus are well known in the art, and utilize, for example homologous recombination. A wide variety of viral promoter sequences, possibly terminator sequences and poly(A)-addition sequences, possibly enhancer sequences and possibly amplification sequences, all required for the mammalian expression, are available in the art. Vaccinia is particularly preferred since vaccinia halts the expression of host cell proteins. Vaccinia is also very much preferred since it allows the expression of f.i. peptides of the present invention in cells or individuals which are immunized with the live recombinant vaccinia virus. For vaccination of humans the avipox and Ankara Modified Virus (AMV) are particularly useful vectors.

Also known are insect expression transfer vectors derived from baculovirus Autographa californica nuclear polyhedrosis virus (AcNPV), which is a helper-independent viral expression vector. Expression vectors derived from this system usually use the strong viral polyhedrin gene promoter to drive the expression of heterologous genes. Different vectors as well as methods for the introduction of heterologous DNA into the desired site of baculovirus are available to the man skilled in the art for baculovirus expression. Also different signals for posttranslational modification recognized by insect cells are known in the art.

The present invention also relates to a host cell transformed with a recombinant vector as defined above.

The present invention also relates to antibodies that are specifically raised against the peptides of the present invention, preferably against those peptides wherein the arginines are derivatized towards citrulline. These antibodies may be polyclonal or monoclonal. To prepare antibodies a host animal is immunized using the peptides of the present invention in a pharmaceutically acceptable carrier, wherein at least one of the arginines is derivatized towards citrulline. Pharmaceutically acceptable carriers include any carrier that does not itself induce the production of antibodies harmful to the individual receiving the composition. Suitable carriers are typically large, slowly metabolized macromolecules such as proteins, polysaccharides, polylactic acids, polyglycolic acids, polymeric amino acids, amino acid copolymers; and inactive virus particles. Such carriers are well known to those of ordinary skill in the art.

Preferred adjuvants to enhance effectiveness of the composition include, but are not limited to : aluminim hydroxide (alum), N-acetyl-muramyl-L-threonyl-D-isoglutamine (thr-MDP) as found in U.S. Patent No. 4,606,918, N-acetylnormuramyl-L-alanyl-D-isoglutamine (nor-MDP), N-acetylmuramyl-L-alanyl-D-isoglutaminyl-L-alanine-2-(1'-2'-dipalmitoyl-sn-glycero-3-hydroxyphosphoryloxy)- ethylamine (MTP-PE) and RIBI, which contains three components extracted from bacteria, monophosphoryl lipid A, trehalose dimycolate, and cell wall skeleton (MPL + TDM + CWS) in a 2% squalene/Tween 80 emulsion. Any of the 3 components MPL, TDM or CWS may also be used alone or combined 2 by 2. Additionally, adjuvants such as Stimulon (Cambridge Bioscience, Worcester, MA) or SAF-1 (Syntex) may be used. Further, Complete Freund's Adjuvant (CFA) and Incomplete Freund's Adjuvant (IFA) may be used for non-human applications and research purposes.

The immunogenic compositions typically will contain pharmaceutically acceptable vehicles, such as water, saline, glycerol, ethanol, etc. Additionally, auxiliary substances, such as wetting or emulsifying agents, pH buffering substances, preservatives, and the like, may be included in such vehicles.

Typically, the immunogenic compositions are prepared as injectables, either as liquid solutions or suspensions; solid forms suitable for solution in, or suspension in, liquid vehicles prior to injection may also be prepared. The preparation also may be emulsified or encapsulated in liposomes for enhanced adjuvant effect. The proteins may also be incorporated into Immune Stimulating Complexes together with saponins, for example Quil A (ISCOMS).

Immunogenic compositions used to raise antibodies comprise a 'sufficient amount' or 'an immunologically effective amount' of the peptides of the present invention, as well as any other of the above mentioned components, as needed. 'Immunologically effective amount', means that the administration of that amount to an individual, either in a single dose or as part of a series, is effective to provoke an immune response and to raise antibodies, as defined above. This amount varies depending upon the health and physical condition of the individual, the taxonomic group of the individual to be treated (e.g. nonhuman primate, primate, rabbit, etc.), the capacity of the individual's immune system to synthesize antibodies, the immunogenicity of the antigenic peptide, and its mode of administration, and other relevant factors. It is expected that the amount will fall in a relatively broad range that can be determined through routine trials. Usually, the amount will vary from 0.01 to 1000 µg/dose, more particularly from 0.1 to 100 µg/dose.

The immunogenic compositions are conventionally administered parenterally, typically by injection, for example, subcutaneously or intramuscularly. Additional formulations suitable for other methods of administration include oral formulations and suppositories. Dosage treatment may be a single dose schedule or a multiple dose schedule. The vaccine may be administered in conjunction with other immunoregulatory agents.

The host serum or plasma is collected following an appropriate time interval to provide a composition comprising antibodies reactive with the peptides of the present invention. The gamma globulin fraction or the IgG antibodies can be obtained, for example, by use of saturated ammonium sulfate or DEAE Sephadex, or other techniques known to those skilled in the art. The antibodies are substantially free of many of the adverse side effects which may be associated with other anti-viral agents such as drugs, for the treatment of infectious, chronic, or recurrent mononucleosis. Such antibodies may also be used to diagnose certain diseases, such as Burkitt's lymphoma, wherein Epstein-Barr virus has been implicated.

The term 'immunogenic' refers to the ability of a substance to cause a humoral and/or cellular response, whether alone or when linked to a carrier, in the presence or absence of an adjuvant.

The antibodies of the claimed invention may also be monoclonals that are prepared with said antibody being specifically reactive with any of said peptides, and with said antibody being preferably a monoclonal antibody.

The monoclonal antibodies of the invention can be produced by any hybridoma liable to be formed according to classical methods from splenic cells of an animal, particularly from a mouse or rat, immunized against the claimed peptides of the present invention on the one hand, and of cells of a myeloma cell line on the other hand, and to be selected by the ability of the hybridoma to produce the monoclonal antibodies recognizing the citrullinated forms of the peptides which has been initially used for the immunization of the animals.

The antibodies involved in the invention can be labelled by an appropriate label of the enzymatic, fluorescent, or radioactive type.

The monoclonal antibodies according to this preferred embodiment of the invention may be humanized versions of mouse monoclonal antibodies made by means of recombinant DNA technology, departing from parts of mouse and/or human genomic DNA sequences coding for H and L chains or from cDNA clones coding for H and L chains.

Alternatively the monoclonal antibodies according to this preferred embodiment of the invention may be human monoclonal antibodies. These antibodies according to the present embodiment of the invention can also be derived from human peripheral blood lymphocytes of patients with rheumatoid arthritis. Such human monoclonal antibodies are prepared, for instance, by means of human peripheral blood lymphocytes (PBL) repopulation of severe combined immune deficiency (SCID) mice (for recent review, see Duchosal et al. 1992) or by screening vaccinated individuals for the presence of reactive B-cells by means of the antigens of the present invention.

The present invention also relates to the anti-idiotype antibodies that are raised upon immunization with an antibody as defined above and that specifically react with said antibodies, thereby mimicking the peptides of the present invention.

The present invention also relates to truncated versions or single chain versions of the antibodies and anti-idiotype antibodies as defined above, that have retained their original specificity for reacting with the antigens.

The present invention also relates to proteins or peptides that mimic the antibodies as defined above such as microproteins as can be obtained by phage display or the highly variable domain of a recombinant antibody as obtained by screening upon repertoire cloning.

The present invention also relates to a method for detecting antibodies that specifically react with the peptides or anti-idiotype antibodies of the present invention, present in a biological sample, comprising:

(i) contacting the biological sample to be analysed for the presence of said antibodies with a peptide or anti-idiotype antibody as defined above,
(ii) detecting the immunological complex formed between said antibodies and said peptide or anti-idiotype antibody.

The present invention also relates to a reverse method for detecting the peptides and/or the anti-idiotype antibodies of the present invention with antibodies present in a biological sample that specifically react with said peptides and/or anti-idiotype antibodies that mimic such peptides, comprising:

(i) contacting the biological sample to be analysed for the presence of said peptides or anti-idiotype antibodies with the antibodies as defined above,
(ii) detecting the immunological complex formed between said antibodies and said peptide or anti-idiotype antibody.

The methods as defined above, can be used in the diagnosis of rheumatoid arthritis.

According to a specific embodiment, the present invention relates to the development of a diagnostic technique that allows differentiation between those autoimmune diseases in which the characteristic antibodies often crossreact with the same antigen, thus resulting in difficult and slow diagnosis. Such diagnostic technique can be obtained by the simultaneous use of several antigens and/or anti-idiotype antibodies of the present invention.

The present invention also relates to a diagnostic kit for use in detecting the presence of said antibodies, said kit comprising at least one peptide or anti-idiotype antibody or microprotein as defined above, with said peptide or anti-idiotype antibody or microprotein being preferably bound to a solid support.

The present invention also relates to a diagnostic kit for determining the type of autoimmune disease, said kit comprising at least one peptide or anti-idiotype antibody or microprotein as defined above, with said peptide or anti-idiotype antibody or microprotein being preferably bound to a solid support.

The present invention also relates to a diagnostic kit as defined above, said kit comprising a range of said peptides and/or anti-idiotype antibodies or microprotein which are attached to specific locations on a solid substrate.

The present invention also relates to a diagnostic kit as defined above, wherein said solid support is a membrane strip and said peptides and/or anti-idiotype antibodies or microproteins are coupled to the membrane in the form of parallel lines.

The immunoassay methods according to the present invention may utilize for instance single or specific oligomeric antigens, dimeric antigens, as well as combinations of single or specific oligomeric antigens. The peptides of the present invention may be employed in virtually any assay format that employs a known antigen to detect antibodies that characterize a certain disease or infection. A common feature of all of these assays is that the antigenic peptide or anti-idiotype antibody or microprotein is contacted with the body component suspected of containing the antibodies under conditions that permit the antigen to bind to any such antibody present in the component. Such conditions will typically be physiologic temperature, pH and ionic strength using an excess of antigen. The incubation of the antigen with the specimen is followed by detection of immune complexes comprised of the antigen.

Design of the immunoassays is subject to a great deal of variation, and many formats are known in the art. Protocols may, for example, use solid supports, or immunoprecipitation. Most assays involve the use of labelled antibody or peptide; the labels may be, for example, enzymatic, fluorescent, chemiluminescent, radioactive, or dye molecules. Assays which amplify the signals from the immune complex are also known; examples of which are assays which utilize biotin and avidin or streptavidin, and enzyme-labelled and mediated immunoassays, such as ELISA assays.

The immunoassay may be, without limitation, in a heterogeneous or in a homogeneous format, and of a standard or competitive type. In a heterogeneous format, the peptide or anti-idiotype antibody or microprotein is typically bound to a solid matrix or support to facilitate separation of the sample from the peptide or anti-idiotype antibody or microprotein after incubation. Examples of solid supports that can be used are nitrocellulose (e.g., in membrane or microtiter well form), polyvinyl chloride (e.g., in sheets or microtiter wells), polystyrene latex (e.g., in beads or microtiter plates, polyvinylidene fluoride (known as Immunolon™), diazotized paper, nylon membranes, activated beads, and Protein A beads. For example, Dynatech Immunolon™ 1 or Immunolon™ 2 microtiter plates or 0.25 inch polystyrene beads (Precision Plastic Ball) can be used in the heterogeneous format. The solid support containing the antigenic peptides or anti-idiotype antibodies or microprotein is typically washed after separating it from the test sample, and prior to detection of bound antibodies. Both standard and competitive formats are know in the art.

In a homogeneous format, the test sample is incubated with the combination of antigens in solution. For example, it may be under conditions that will precipitate any antigen-antibody or anti-idiotype antibody-antibody or microprotein-antibody complexes which are formed. Both standard and competitive formats for these assays are known in the art. For instance, to characterize rheumatoid arthritis in a standard format, the amount of rheumatoid arthritis antibodies in the antibody-antigen complexes is directly monitored. This may be accomplished by determining whether a second type of labelled anti-xenogenetic (e.g. anti-human) antibodies which recognize an epitope on the first type of rheumatoid arthritis-antibodies will bind due to complex formation. In a competitive format, the amount of rheumatoid arthritis-antibodies in the sample is deduced by monitoring the competitive effect on the binding of a known amount of labelled antibody (or other competing ligand) in the complex. The detection of rheumatoid arthritis-antibodies for diagnosis of rheumatoid arthritis is used as an illustration. Wherever the term "rheumatoid arthritis-antibodies" is used throughout the specification, this should not be considered as limitative. Like wise, the other autoimmune diseases are diagnosed by detection of other antibodies, and mononucleosis is diagnosed by detection of anti-Epstein-Barr virus antibodies.

Complexes formed comprising rheumatoid arthritis-antibody (or in the case of competitive assays, the amount of competing antibody) are detected by any of a number of known techniques, depending on the format. For example, unlabelled rheumatoid arthritis-antibodies in the complex may be detected using a conjugate of anti-xenogenetic Ig complexed with a label (e.g. an enzyme label).

In an immunoprecipitation or agglutination assay format the reaction between the rheumatoid arthritis-antigens and the rheumatoid arthritis-antibody forms a network that precipitates from the solution or suspension and forms a visible layer or film of precipitate. If no rheumatoid arthritis-antibody is present in the test specimen, no visible precipitate is formed.

Currently, there exist three specific types of particle agglutination (PA) assays. These assays are used for the detection of antibodies to various antigens when coated to a support. One type of this assay is the hemagglutination assay using red blood cells (RBCs) that are sensitized by passively adsorbing antigen (or antibody) to the RBC. The addition of specific antigen antibodies present in the body component, if any, causes the RBCs coated with the purified antigen to agglutinate.

To eliminate potential non-specific reactions in the hemagglutination assay, two artificial carriers may be used instead of RBC in the PA. The most common of these are latex particles. However, gelatin particles may also be used.

The assays utilizing either of these carriers are based on passive agglutination of the particles coated with purified antigens.

The antigenic peptides of the present invention will typically be packaged in the form of a kit for use in these immunoassays. The kit will normally contain in separate containers the antigenic peptide or anti-idiotype antibody, control antibody formulations (positive and/or negative), labeled antibody when the assay format requires the same and signal generating reagents (e.g. enzyme substrate) if the label does not generate a signal directly. The antigenic peptide or anti-idiotype antibody may be already bound to a solid matrix or separate with reagents for binding it to the matrix. Instructions (e.g. written, tape, CD-ROM, etc.) for carrying out the assay usually will be included in the kit.

The solid phase selected can include polymeric or glass beads, nitrocellulose, microparticles, microwells of a reaction tray, test tubes and magnetic beads. The signal generating compound can include an enzyme, a luminescent compound, a chromogen, a radioactive element and a chemiluminescent compound. Examples of enzymes include alkaline phosphatase, horseradish peroxidase and beta-galactosidase. Examples of enhancer compounds include biotin, anti-biotin and avidin Examples of enhancer compounds binding members include biotin, anti-biotin and avidin. In order to block the effects of rheumatoid factor-like substances, the test sample is subjected to conditions sufficient to block the effect of rheumatoid factor-like substances. These conditions comprise contacting the test sample with a quantity of for instance Rabbit Ig or anti-human IgG, preferably aggregated, to form a mixture, and incubating the mixture for a time and under conditions sufficient to form a reaction mixture product substantially free of rheumatoid factor-like substance.

The present invention particularly relates to an immunoassay format in which several peptides of the invention are coupled to a membrane in the form of parallel lines. This assay format is particularly advantageous for allowing a discrimination between the separate autoimmune diseases.

**Legends to the Figures**

Fig 1: HPLC profile of tryptic digests of human natural acidic (a) and neutral (b) filaggrin.
Peptides were separated by reversed-phase HPLC on a C-4 Vydac column (2.1 x 250 mm, Hesperia, CA) using a 140B Solvent Delivery System (ABI, Foster City, CA) and eluted with a 8-70% linear gradient of 70% acetonitrile in 0.1 % trifluoroacetic acid (TFA). Detection occured at 214 nm with a 1000S diode array detector.

Fig 2. Multiple alignment of filaggrin protein sequences and overview of the sequenced peptides. HB2641, HB2642, HB2648 and HB2650 clones were isolated in house; all other sequences were retrieved from literature.
Character to show that a position is perfectly conserved: '*'
Character to show that a position is well conserved: '.'

Fig3: Reactivity of 26 human RA sera with synthetic peptides on LIA.
Peptides IGP1155, 1156, 1157 and 1158 all contained citrulline; IGP1179, 1180, 1181 and 1182 were the corresponding counterparts without citrulline incorporated. Peptides were applied after complexing with streptavidin at a concentration of 400 μg/ml. IGP1154 consisted of an irrelevant synthetic peptide.

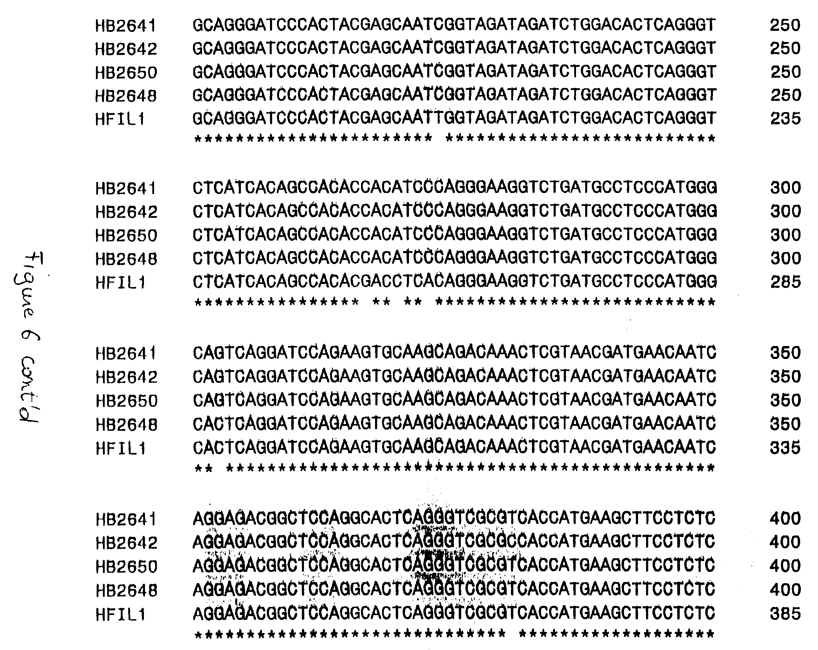

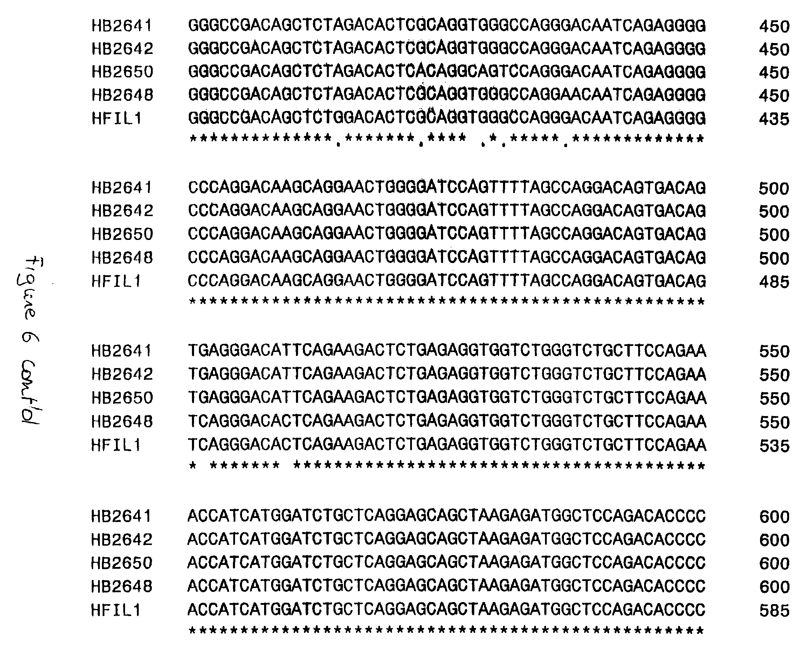

Fig4: Inhibition ELISA using natural filaggrin as inhibitory agent.
Plates were coated with purified human natural filaggrin at 1 μg/ml. Sera were diluted 1/50 and added to the plate with or without preincubation with natural filaggrin at 1 or 10 μg/ml. OD values were measured at 450 nm.

Fig5: Inhibition ELISA using synthetic citrulline-containing filaggrin peptides as inhibitory agents.
Plates were coated with purified human natural filaggrin at 4 μg/ml. Sera were diluted 1/50 and added to the plate with or without preincubation with the individual peptides (IGP1155, 1156, 1157, 1158) or a mixture of the four peptides (mix), each at 100 μg/ml. OD values were measured at 450 nm.

Fig 6: Multiple sequence alignment of clones HB2641, HB2642, HB2648 and HB2650 with the HFIL1 sequence retrieved from literature (McKinley-Grant et al. 1989).
Character to show that a position is perfectly conserved: '*'
Character to show that a position is well conserved: '*'

**Examples**

<u>**Example 1: Epitope mapping of filaggrin**</u>

1.1 Sera

Human sera were obtained from the Department of Rheumatology of the University Hospital in Ghent (Belgium). In total, 265 sera were included, of which 75 fulfilled the ARA criteria for RA (Arnett et al., Arthritis Rheum.,31: 315-324, 1987), 155 sera scored positive in the APF fluorescence test (De Keyser et al., in press), 98 reacted with natural filaggrin on Western blot, 80 were APF negative and 16 were derived from healthy controls.

1.2 Preparation of human filaggrin

Natural filaggrin was purified from human skin obtained freshly after abdominoplasty according to the protocol of Simon et al. (J. Clin. Invest., 92: 1387-1393. 1993). The epidermis was separated from the dermis by incubating the skin pieces at 56°C in PBS containing 5 mM EDTA. The material was stored dry at -20°C untill use. The epidermis was cut into small pieces which were homogenized in 40 mM Tris-HCl, pH 7.4, 150 mM NaCl, 5 mM EDTA, 0.5% NP-40, 0.1% sodium azide, 0.1 mM PMSF ($0.2$ ml/cm$^2$) and stirred overnight at 4°C. The homogenate was centrifuged at 15,000 x g for 15 min and the extracted proteins in the supernatant were precipitated overnight at -20°C by adding 5 volumes of absolute ethanol. After centrifugation for 15 min at 10,000 x g, the protein pellet was vacuum-dried and subsequently resuspended in water. This partially purified protein preparation is refered to filaggrin used in the present study. The amount of protein was determined by the Bradford protein assay as modified by Peterson (Methods Enzymol., 91: 108-110. 1983) using BSA standard curves.

1.3 Immune detection of antifilaggrin antibodies in human sera

The crude filaggrin preparation was submitted to 10% Tricine SDS-PAGE using the Bio-Rad mini-gel apparatus. Two µg protein/cm was loaded in a large slot and electrophoresed under standard conditions. The gel was subsequently electroblotted onto nitrocellulose membrane in 10% methanol, 10 mM CAPS, pH 11.0 during 40 min. The blot was blocked in PBS, 0.05% Tween20, 1% gelatin and cut into 3 mm strips, which were probed with human sera overnight at a 1/100 dilution in PBS, 0.05% Tween20, 0.1% gelatin. As a secondary antibody the anti-human IgG-AP conjugate (Sigma, St Louis, MI) was added at a 1/1000 dilution; visualization occured with the NBT/BCIP chromogenic substrate.

1.4 Two-dimensional electrophoresis of filaggrin

The filaggrin material was separated by 2-D electrophoresis using Immobiline DryeStrips pH 3-10 (Pharmacia Biotech, Uppsala, Sweden) in the first dimension and 10% Tricine SDS-PAGE in the second dimension using standard procedures. Preparative gels were loaded with 300 µg protein and stained with Coomassie R-250. The large comma-shaped filaggrin spot was identified by immunoreaction with the antifilaggrin mAb (BTI, Stoughton, MA). The pI of this heterogeneous protein ranged from 6.7-8.5 (7.1-8.5 on gel), while molecular weight forms of 35-68 kDa were detected, with the more acidic isoforms representing the highest masses.

1.5 Electro-elution of filaggrin

The large filaggrin spot was cut out of the gel and separated into three parts: i) acidic fraction with pI 7.1-7.5; ii) neutral fraction with pI 7.5-8.1; iii) basic fraction with pI 8.1-8.5. Each fraction was electro-eluted by the method of Hunkapiller et al. (Methods Enzymol., 91: 227-236. 1983) using 50 mM $(NH_4)HCO_3$, 0.1 % SDS as elution buffer. Coomassie stain and SDS were removed from the eluted proteins by ion pair extraction as described by Königsberg and Henderson (Methods Enzymol., 91: 254-259. 1983). Vacuum-dried protein pellets were redissolved in the appropriate buffer for further analysis.

1.6 Peptide mapping

Purified electro-eluted filaggrin fractions of ± 100 µg were dissolved in 40 µl 100 mM $(NH_4)HCO_3$, pH 8.0, 10% acetonitrile and digested with trypsin (1/40 E/S ratio). After overnight incubation at 37°C the digest was stored at -20°C before use.
Peptides were separated by reversed-phase HPLC on a C-4 Vydac column (2.1 x 250 mm, Hesperia, CA) using a 140B Solvent Delivery System (ABI, Foster City, CA) and eluted with a 8-70% linear gradient of 70% acetonitrile in 0.1% trifluoroacetic acid (TFA). Detection occured at 214 nm with a 1000S diode array detector and peptides were manually recovered.

1.7 Dot spot analysis and microsequencing

Ninety percent of each peak fraction was vacuum-dried and subsequently resuspended in a small volume of 10% acetonitrile, 50 mM NaCO$_3$ buffer, pH 9.5. The peptides were dot spotted onto Immunodyne ABC membranes (Pall BioSupport, UK), which were probed with human sera in order to assign the immunoreactive epitopes. First, membranes were blocked with PBS, 0.5% caseine and incubated overnight with sera 1/50 diluted in PBS, 0.5% caseine, 0.1 % Triton X705, 10 mM MgCl$_2$.6H$_2$O. After washing with PBS, 0.05% Tween20, anti-human IgG (Promega) diluted in PBS, 0.1% caseine, 0.2% Triton X705 was added for 1h30. Membranes were developed in 100 mM NaCl, 100 mM Tris-HCl, pH 9.8, 50 mM MgCl$_2$.6H$_2$O substrate buffer containing the chromogenic substrate NBT/BCIP. Reaction was stopped by addition of 0.2 N H$_2$SO$_4$.
The remaining 10% of the immunoreactive fractions was used for microsequencing. Therefore, fractions were directly analyzed on a pulsed-liquid model 477A Sequencer equipped with an on-line 120 phenylthiohydantoin analyser (ABI).

1.8 Results

Acidic filaggrin

Each fraction of the tryptic digest of acidic filaggrin (Fig 1a) was dot spotted in triplicate on ABC membranes. Four APF positive sera reacting with human filaggrin on Western blot (IG24395, IG35247 and IG24183/24184 pool) and one APF negative control serum were used for immunoreaction. Fractions T2, T9, T16 and T66 showed weak reaction with the IG24395 serum, while the series from T30 to T44, especially T34 and T38 peptide fractions, showed clear positive reaction. The APF serum pool also scored positive with both T34 and T38, while IG35247 was only reactive with T4. The APF negative control serum was clearly unreactive with the peptide fractions.
Following amino acid sequences were retrieved in each fraction:

T2:
R↓AGHGHSADSSR
T4:
R↓QGSRHQQAR
R↓AGHGHSADSSR
R↓HGSHHQQSADSSR
T9:
R↓HSQVGQGESSGPR
T16
R↓HSASQDGQDTIRGHPG
T33:
R↓HSASQDGQDTIRGH
R↓HSGIGHGQASSAVR
T34:
R↓HSASQDGQDTI
T35:
R↓HSGIGHGQASSAVR
R↓DSGHRGYSGSQASDNEGH
R↓HSTSQEGQDTIHGHRGS
R↓HSASQDGQDTIRGHPG
T38; T39:
Same peptides as in fraction T35
T40, T41:
Same peptide as in fraction T34
T66:
No signals could be retrieved.

R represents the amino acid citrulline, which was found at a specific retention time different from that of arginine. Further proove for the presence of citrulline was i) the fact that the sequence was not cleaved at that specific position, which would be expected if arginine was present and ii) that no arginine residue was sequenced.

Neutral filaggrin

A similar procedure was carried out for neutral filaggrin (Fig 1b) and dot spots were incubated with APF sera IG24395 and IG24184 and the same negative control serum as used for the acidic filaggrin mapping. Fractions T28, T65, T81 and the series from T30 towards T40 (especially T32, T35 and T36) reacted positively with one APF serum IG24395. The other serum showed weak reaction with T4, T28, T45 and T81. There was one fraction (T48) that showed aspecific reactivity with all sera including the negative control serum.
Following sequencing results were retrieved:

T32, T36, T37, T48 and T65 yielded no reliable amino acid sequencing signals.
T35:
R↓HSGIGHGQASSAVR
R↓DSGHRGYSGSQASDNEGH
R↓HSTSQEGQDTIHGHRGS
R↓HSASQDGQDTIRGHPG
R↓GYSGSQASDNEGHSE

The fifth peptide was clearly derived from the second peptide, most probably due to the fact that in the neutral filaggrin two forms with and without citrulline-modification do exist. These results indicate that the same four peptides were retrieved as identified in the acidic T35 fraction.

Second mapping of neutral filaggrin

A mix of two other sera IG35038/ 35041 that were both negative on the synthetic peptides IGP1155, 1156, 1157 or 1158 (see example 2) was used for mapping with peptides of neutral filaggrin derived from another skin source. This yielded specific reactivity with fractions T28, T31, T34 and T35, while the APF negative serum showed no reaction. Sequencing results:

T28, T31, T34 yielded no sequencable signals.
T35:
R↓NDEQSGDGSR

An overview of the sequenced immunoreactive parts of filaggrin molecules is shown in Fig 2.

**Example 2: Reactivity of synthetic peptides in a Line Immuno Assay (LIA) system**

2.1 Synthetic peptides

Amino acid sequencing results described in example 1 were used for the generation of synthetic peptides. For some citrulline-containing peptides, the non-modified counterpart with arginine was synthesized in order to compare their respective reactivities. The following peptides were made.

| IGP1155 | IGP1179 |
|---|---|
| HSASQDGQDTIRGHPGSS | HSASQDGQDTIRGHPGSS |
| IGP1156 | IGP1180 |
| HSGIGHGQASSAVRDSGHRGYS | HSGIGHGQASSAVRDSGHRGYS |
| IGP1157 | IGP1181 |
| DSGHRGYSGSQASDNEGH | DSGHRGYSGSQASDNEGH |
| IGP1158 | IGP1182 |
| HSTSQEGQDTIHGHRGS | HSTSQEGQDTIHGHRGS |

(continued)

| IGP1249 | |
| GGQGS$\underline{\underline{R}}$HQQAR | |
| IGP1250 | |
| GGAGHGHSADSSR | |
| IGP1251 | |
| GGHGSHHQQSADSSR | |
| IGP1252 | |
| GGNDEQSGDGSRHSGS | |
| IGP1326 | |
| SRHSQVGQGESSGPR | |
| $\underline{\underline{R}}$ represents citrulline | |

## 2.2 Line Immuno Assay analysis

Streptavidin-complexed peptides were applied directly on a nylon membrane with a plastic backing. Blocked strips were incubated overnight with human sera diluted 1/100 in 1 ml PBS, 0.5% caseine, 0.1% Triton X705, 10 mM MgCl$_2$.6H$_2$O. After washing with PBS, 0.05% Tween20, goat anti-human IgG-AP conjugated (Promega) diluted in PBS, 0.1% caseine, 0.2% Triton X705 was added for 1h30. Strips were developed in 100 mM NaCl, 100 mM Tris-HCl, pH 9.8, 50 mM MgCl$_2$.6H$_2$O substrate buffer containing the chromogenic substrate NBT/ BCIP. Reaction was stopped after 30 min by addition of 0.2 N H$_2$SO$_4$.

## 2.3 Results

A group of 75 sera derived from patients who fulfilled the ARA criteria for RA were tested in the LIA system for reactivity with the 13 synthetic peptides listed in 2.1. Reactivity was noted with IGP1155, 1156, 1157, 1158 and 1249 (Table 2), with a combined sensitivity of 50% in this group. Analysis of 155 APF positive sera, which consisted of 61 RA's and 94 sera with unknown diagnosis, yielded highly comparable reaction. Taking into account only those sera that reacted with human natural filaggrin on Western blot, higher sensitivities were reached for each synthetic peptide. When calculating the reactivity of these sera with either one of the peptides, a sensitivity of 70% was obtained, while only 5 out of 80 APF negative sera and 2 healthy controls scored positive. In all groups, IGP1156 and 1158 appeared the best reactive peptides, while immunoreaction towards IGP1157 was always very faint and did not add any value in terms of increasing the sensitivity for RA diagnosis.

The relationship between filaggrin postivity on Western blot and reactivity with the synthetic peptides within the RA group is depicted in Table 3. Upon statistical analysis, a high correlation was observed between both reactivity patterns (kappa value is 0.6; agreement coefficient is 80 %).

The positive LIA signals observed with the citrulline-containing peptides IGP1155, IGP1156, IGP1157, and IGP1158 disappeared completely when tested with the non-modified counterparts IGP1179, IGP1180, IGP1181, and IGP1182 (Fig. 3). Only for 2 sera, a weak colouring of all non-modified peptides was obtained, which could be regarded as aspecific background staining related to the particular sera.These results indicate that the presence of citrulline is indispensable for immunoreactivity and that this unusual amino acid constitutes an important epitope for antifilaggrin antibodies.

## Example 3: Reactivity of synthetic peptides in ELISA

### 3.1 ELISA

Electro-eluted human natural filaggrin was coated in Maxisorp polystyrene plates at a concentration of 1 or 4 µg/ml in 50 mM carbonate buffer, pH 9.6 overnight at 4°C. Sera were diluted 1/50 in PBS, and preincubated with either the appropriate peptides at 100 µg/ml, with isolated natural filaggrin at 1 or 10 µg/ml (positive control) or with PBS (negative control) for 2 hours. The plates were blocked 1 hour with PBS, 0.1% caseïne at 37°C and subsequently incubated with the sera. During 2 hours at 37°C. Plates were washed 5 times with PBS, 0.05% Tween 20 and incubated with anti-human IgG-HRP in PBS, 0.1 % caseïne, 0.1 mM K$_3$Fe(CN)$_6$. Colour development was performed using tetramethylbenzidine diluted 1/100 into 0.1 M $Na_2HPO_4$, 0.1 M citric acid, 0.006% $H_2O_2$, pH 4.3. Reaction was stopped by addition of 2N $H_2SO_4$ and OD values were measured at 450 nm using a Bio-tek ELISA reader.

The percentage of inhibition was caculated as folows:

$$\frac{(\text{OD without inhibition} - \text{OD with inhibition})}{\text{OD without inhibition}} \times 100$$

3.2 Results

Five APF positive sera showing strong immunoreactivity towards natural filaggrin on blot were analyzed on filaggrin-coated plates, with and without inhibitory filaggrin added (Fig 4; Table 4). Upon preincubation at a concentration of 10 μg/ml, the signal dropped with 64-79%, which was significantly higher than with the use of 1 μg/ml (4-45%). The APF negative serum IG24805 not reactive with filaggrin in Western blot yielded only background signals in this ELISA and no significant inhibition could be observed.

When using either one of the synthetic, citrulline-containing peptides IGP1155, 1156, 1157, 1158 or a mixture of these four peptides as competitor, each at a concentration of 100 μg/ml, 2 out of 4 sera could be significantly inhibited by IGP1155 and 1 serum by IGP1158, which was in agreement with the LIA results (Fig 5; Table 4). The peptide mixture was able to inhibit the anti-filaggrin binding of the four sera to an extend of 37-92%, while no inhibition was observed with the APF negative serum.

**Example 4: Cloning and expression of human filaggrin**

Four candidate human filaggrin sequences were cloned using PCR technology, using genomic DNA isolated from human lymphocytes as template. PCR primers were designed as follows:

Sense PCR primer:

              **EcoRI**

5' CC **GAA TTC** GCC ACC ATG <u>GGG TCT TTC CTC TAC CAG GTG</u> 3'

                        Met <u>Gly   Ser   Phe   Leu   Tyr   Gln   Val</u>

The PCR sense primer was chosen to overlap the filaggrin linker sequence and was designed to introduce a functional initiation codon (Kozak environment) upstream of the linker sequence (Phe Leu Tyr Gln Val Ser Thr). The linker sequence was included in the amplified filaggrin repeat, because it is possibly involved in the correct targetting of the processed protein. An EcoRI restriction site was introduced for subcloning of the PCR fragment.

The antisense PCR primer:

      <u>Thr   Ser   Gly   His   Ser   Gly   Ser</u>   Pro   Gly   His   STOP

3' <u>TGC AGA CCT GTA AGT CCT AGA</u> **GGG CCC GAG ATC TGG** 5'

                          **SmaI**         **XbaI**

The antisense primer is located just upstream from the next filaggrin linker sequence and introduces a translation stop codon TAG. The amplified filaggrin sequence consists therefore of the filaggrin linker followed by an integral filaggrin repeat and three additional amino acids (Pro/Gly/His) resulting from the cloning strategy. The PCR amplified fragments were cloned in a pBLSK (Stratagene) vector, as EcoRI-XbaI fragment, allowing sequencing of the amplified cDNA.

Table 2

| Immunoreactivity of human sera with 5 different citrulline-containing synthetic peptides. | | | | | | | |
|---|---|---|---|---|---|---|---|
| Serum group | Number of sera | IGP1155 | IGP1156 | IGP1157 | IGP1158 | IGP1249 | Combination |
| APF pos. | 155 | 35 (22.6%) | 50 (32.2%) | 4/128 (3.1%) | 42 (27.1%) | 23/150 (15.3%) | 80 (51.6%) |
| RA sera | 75 | 17 (22.7%) | 25 (33.3%) | 3/72 (4.2%) | 24 (32%) | 9/73 (12.3%) | 38 (50.7%) |
| Fg blot pos. | 98 | 35 (37.2%) | 46 (48.9%) | 4/94 (4.2%) | 41 (43.6%) | 17/94 (18.1%) | 69 (70.4%) |
| APF neg. | 80 | 4 (5.5%) | 4 (5.5%) | 2/61 (3.3%) | 5/79 (6.3%) | 1/79 (1.3%) | 5 (6.3%) |
| Healthy | 16 | 1 (6.3%) | 2 (12.5%) | 0/10 (0%) | 1 (6.3%) | 1 (6.3%) | 2 (12%) |

The four individual clones characterized by sequencing were named HB2641, HB2642, HB2648 and HB2650 (Fig 6) . The cDNA inserts were recloned as EcoRV/Ecl 136II fragments (1030 bp) in the E. coli expression vector pIGRHISA opened with HsiI blunted. The filaggrin proteins were expressed as recombinant filaggrin-His6 fusion proteins in three different E. Coli strains, resulting in high Coomassie stainable expression levels. The His6 tail of the fusion protein allows easy purification of the protein using metal-affinity chromatography.

Table 3

| Correlation between anti-filaggrin positivity on Western blot and reactivity with 5 synthetic peptides IGP1155, 1156, 157, 1158 and 1249. | | | |
|---|---|---|---|
| RA sera | | LIA- | combined |
| (N=75) | | + | - |
| Filaggrin blot | + | 31 | 8 |
| | - | 7 | 29 |

**List of References**

Arnett F.C., Edworthy S.M., Bloch D.A., McShane D.J., Fries J.F., Cooper N.S., Healy L.A., Kaplan S.R., Liang M.H., Luthra H.S., Medsger T.A., Mitchell D.M., Neustadt D.H., Pinals R.S., Schaller J.G., Sharp J.T., Wilder R.L., Hunder G.G. (1987) The American Rheumatism Association 1987 revised criteria for the classification of rheumatoid arthritis. Arthritis Rheum., 31: 315-324

Chen P.P., Fong S., Carson D.A. (1987) Rheumatoid factor. Rheum. Dis. North Am., 13: 545-568

Dale B.A., Holbrook K.A., Steinert P.M. (1978) Assembly of stratum corneum basic protein and keratin filaments in macrofibrils. Nature, 276: 729-731

Feltkamp T.E., Berthelot J.M., Boerbooms A.M., Geertzen H.G., Hoet R., De Keyser F. (1993) Interlaboratory variability of the antiperinuclear factor (APF) test for rheumatoid arthritis. Clin. Exp. Rheumatol., 11: 57-59

Gan S.Q., McBride O.W., Idler W.W., Markova N., Steinert P.M. (1990) Organization, structure, and polymorphisms of the human profilaggrin gene. Biochemistry, 29: 9432-9440

**Hajiroussou V.J., Skingle J., Gillett A.P., Webley M. (1985)** Significance of antikeratin antibodies in rheumatoid arthritis. J. Rheumatol., 12: 57-59

**Harding C.R., Scott I.R. (1983)** Histidine-rich proteins (filaggrins): structural and functional heterogeneity during epidermal differentiation. J. Mol. Biol., 170: 651-673

**Hassfeld W., Steiner G., Graninger W., Witzmann G., Schweitzer H., Smolen J.S. (1993)** Autoantibodies to the nuclear antigen RA33: a marker for early rheumatoid arthritis. Br. J. Rheumatol., 32: 199-203

**Janssens X., Veys E.M., Verbruggen G., Declercq L. (1988)** The diagnostic significance of the antiperinuclear factor for rheumatoid arthritis. J. Rheumatol., 15: 1364- 1350

**Johnson G.D., Carvalho A., Holborow E.J., Goddard D.H., Russell G. (1981)** Antiperinuclear factor and antikeratin antibodies in rheumatoid arthritis. Ann. Rheum. Dis., 40: 263-266

**Kataaha P.K., Mortazavi-Milani S.M., Russell G., Holborow E.J. (1985)** Anti-intermediate filament antibodies, antikeratin antibodies, and perinuclear factor in rheumatoid arthritis and infectious mononucleosis. Ann. Rheum. Dis., 44: 446-449

**Kirstein H., Hjarvard K., Moørk Hansen T.( 1989)** Antikeratin antibodies in synovial fluid in rheumatoid arthritis. Acta Pathol. Microbiol. Scand., 97: 185-189

**Köningsberg W.H., Henderson L. (1983)** Removal of Sodium Dodecyl Sulfate from proteins by ion-pair extraction. Meth. Enzymol., 91: 254-259

**Lynley A.M., Dale B.A. (1983)** The caracterization of human epidermal filaggrin, a histidine-rich, keratin filament-aggregating protein. Biochem. Biophys. Acta, 744: 28-35

**Mack J.W., Steven A.C., Steinert P.M. (1993)** The mechanism of interaction of filaggrin with intermediate filaments. J. Mol. Biol., 232: 50-66

**Maniatis T., Fritsch E., Sambrook J. (1982)** Molecular cloning: a laboratory manual. Cold Spring Harbor Laboratory Press, Cold Spring Harbor, NY.

**Markova N.G., Marekov L.N., Chipev C.C., Gan S.-Q., Idler W.W., Steinert P.M. (1993)** Profilaggrin is a major epidermal calcium-binding protein. Mol. Cell. Biol., 13: 613-625

**McKinley-Grant L.J., Idler W.W., Bernstein I.A., Parry D.A.D., Cannizzaro L., Croce C.M., Huebner K., Lessin S.R., Steinert P.M. (1989)** Characterization of a cDNA clone encoding human filaggrin and localization of the gene to chromosome region 1q21. Proc. Natl. Acad. Sci. USA, 86: 4848-4852

**Miossec P., Youinou P., Le Goff P., Moineau M.P. (1982)** Clinical relevance of antikeratin antibodies in rheumatoid arthritis. Clin. Rheumatol., 1: 185-189

**Nesher G., Moore T.L., Gristani M.W., El-Najdawi E., Osborn T.G. (1992)** Antiperinuclear factor in juvenile rheumatoid arthritis. Ann. Rheum. Dis., 51: 350-352

**Nienhuis R.L.F., Mandema E. (1964)** A new serum factor in patients with rheumatoid arthritis. The antiperinuclear factor. Ann. Rheum. Dis., 23: 302-305

**Peterson G.L. (1983)** Determination of total protein. Meth. Enzymol., 91: 95-119

**Presland R.B., Haydock P.V., Fleckman P., Nirunsuksiri W., Dale B.A. (1992)** Genomic organization and identification of an S-100-like calcium binding domain at the amino terminus. J. Biol. Chem., 267: 23772-23781

**Resing K.A., Walsh K.A., Haugen-Scofield J., Dale B.A. (1989)** Identification of proteolytic cleavage sites in the conversion of profilaggrin to filaggrin in mammalian epidermis. J. Biol. Chem., 264: 1837-1845

**Resing K.A., Johnson R.S., Walsh K.A. (1993)** Characterization of protease sites during conversion of rat profilaggrin to filaggrin. Biochemistry, 32: 10036-10045

**Scott I.R., Harding C.R., Barrett J.G. (1982)** Histidine-rich protein of the keratohyalin granules. Source of the free amino acids, urocanic acid and pyrrolidone carboxylic acid in the stratum corneum. Biochem. Biophys. Acta, 719: 110-117

**Sebbag M., Simon M., Vincent C., Masson-Bessière C., Girbal E., Durieux J.-J., Serre G. (1995)** The antiperinuclear factor and the so-called antikeratin antibodies are the same rheumatoid arthritis-specific autoantibodies. J. Clin. Invest., 95: 2672-2679

**Simon M., Girbal E., Sebbag M., Gomès-Daudrix V., Vincent C., Salama G., Serre G. (1993)** The cytokeratin filament-aggregating protein filaggrin is the target of the so-called 'antikeratin antibodies', autoantibodies specific for rheumatoid arthritis. J. Clin. Invest., 92: 1387-1393

**Simon M., Vincent C., Haftek M., Girbal E., Sebbag M., Gomès-Daudrix V., Serre G. (1995)** The rheumatoid arthritis-associated autoantibodies to filaggrin label the fibrous matrix of the cornified cells but not the profilaggrin-containing keratohyalin granules in human epidermis. Clin. Exp. Immunol., 100: 90-98

**Vincent C., Serre G., Lapeyre F., Fournié B., Ayrolles C., Fournié A., Soleilhavoup J.-P. (1989)** High diagnostic value in rheumatoid arthritis of antibodies to the stratum corneum of rat oesophagus epithelium, so-called 'antikeratin antibodies'. 1989. Ann. Rheum. Dis., 48: 712-722

**Vincent C., Serre G., Basile J.-P., Lestra H.C., Girbal E., Sebbag M., Soleilhavoup J.-P. (1990)** Subclass distribution of IgG antibodies to the rat oesophagus stratum corneum (so-called anti-keratin antibodies) in rheumatoid arthritis. Clin. Exp. Immunol., 81: 83-89

**Vincent C., De Keyser F., Veys E.M., Serre G. (1997)** Comparative study of APF, AKA and anti-filaggrin autoantibodies in rheumatoid arthritis and other arthritides. Ann. Med. Interne, 148: 52

**Vivino F.B., Maul G. G. (1989)** Antiperinuclear factor prevalence for rheumatoid arthritis. Arthritis Rheum., 32: S95

**Vivino F.B., Maul G.G. (1990)** Histologic and electron microscopic characterization of the antiperinuclear factor antigen. Arthritis Rheum., 33: 960-969

**Waller M.V., Toone E.C., Vaughan E. (1964)** Study of rheumatoid factor in a normal population. Arthr. Rheum., 7: 513-520

**Westgeest A.A.A., Boerbooms A.M.Th., Jongmans M., Vandenbroucke J.P., Vierwinden G., van de Putte L.B.A. (1987)** Antiperinuclear factor: Indicator of more severe disease in seronegative rheumatoid arthritis. J. Rheumatol., 14: 893-897

**Youinou P., Le Goff P., Colaco C.B., Thivolet J., Tater D., Viac J., Shipley M. (1985)** Antikeratin antibodies in serum and synovial fluid show specificity for rheumatoid arthritis in a study of connective tissue diseases. Ann. Rheum. Dis., 44: 450-454

**Youinou P., Berthelot J.M., Peron A., Leroux A.M., Le Goff P. (1992)** Antiperinuclear antibodies and corresponding antigens. Rev. Rhum. Mal. Osteoartic., 59: 43S-51S

**Young B.J.J, Mallya R.K., Leslie R.D.G., Clark C.J.M., Hamblin T.J. (1979)** Anti-keratin antibodies in rheumatoid arthritis. Br. Med. J., 2: 97-99

SEQUENCE LISTING

(1) GENERAL INFORMATION:

    (i) APPLICANT:
        (A) NAME: Innogenetics N.V.
        (B) STREET: Industriepark Zwijnaarde 7, box 4
        (C) CITY: Ghent
        (E) COUNTRY: Belgium
        (F) POSTAL CODE (ZIP): B-9052
        (G) TELEPHONE: 00 32 9 241 07 11
        (H) TELEFAX: 00 32 9 241 07 99

    (ii) TITLE OF INVENTION: Synthetic peptides containing citrulline
        recognized by rheumatoid arthritis sera as tools for
        diagnosis and treatment.

    (iii) NUMBER OF SEQUENCES: 49

    (iv) COMPUTER READABLE FORM:
        (A) MEDIUM TYPE: Floppy disk
        (B) COMPUTER: IBM PC compatible
        (C) OPERATING SYSTEM: PC-DOS/MS-DOS
        (D) SOFTWARE: PatentIn Release #1.0, Version #1.30 (EPO)

    (v) CURRENT APPLICATION DATA:
        APPLICATION NUMBER: EP 98870078.7

(2) INFORMATION FOR SEQ ID NO: 1:

    (i) SEQUENCE CHARACTERISTICS:
        (A) LENGTH: 18 amino acids
        (B) TYPE: amino acid
        (C) STRANDEDNESS: single
        (D) TOPOLOGY: linear

    (ii) MOLECULE TYPE: peptide

    (iii) HYPOTHETICAL: NO

    (iv) ANTI-SENSE: NO

    (xi) SEQUENCE DESCRIPTION: SEQ ID NO: 1:

    His Ser Ala Ser Gln Asp Gly Gln Asp Thr Ile Arg Gly His Pro Gly
    1            5                  10                15

    Ser Ser

(2) INFORMATION FOR SEQ ID NO: 2:

    (i) SEQUENCE CHARACTERISTICS:
        (A) LENGTH: 22 amino acids
        (B) TYPE: amino acid

```
                (C) STRANDEDNESS: single
                (D) TOPOLOGY: linear

        (ii) MOLECULE TYPE: peptide

        (iii) HYPOTHETICAL: NO

        (iv) ANTI-SENSE: NO




        (xi) SEQUENCE DESCRIPTION: SEQ ID NO: 2:

        His Ser Gly Ile Gly His Gly Gln Ala Ser Ser Ala Val Arg Asp Ser
        1               5                   10                  15

        Gly His Arg Gly Tyr Ser
                    20

(2) INFORMATION FOR SEQ ID NO: 3:

        (i) SEQUENCE CHARACTERISTICS:
            (A) LENGTH: 18 amino acids
            (B) TYPE: amino acid
            (C) STRANDEDNESS: single
            (D) TOPOLOGY: linear

        (ii) MOLECULE TYPE: peptide

        (iii) HYPOTHETICAL: NO

        (iv) ANTI-SENSE: NO




        (xi) SEQUENCE DESCRIPTION: SEQ ID NO: 3:

        Asp Ser Gly His Arg Gly Tyr Ser Gly Ser Gln Ala Ser Asp Asn Glu
        1               5                   10                  15

        Gly His


(2) INFORMATION FOR SEQ ID NO: 4:

        (i) SEQUENCE CHARACTERISTICS:
            (A) LENGTH: 17 amino acids
            (B) TYPE: amino acid
            (C) STRANDEDNESS: single
            (D) TOPOLOGY: linear

        (ii) MOLECULE TYPE: peptide

        (iii) HYPOTHETICAL: NO

        (iv) ANTI-SENSE: NO
```

```
(xi) SEQUENCE DESCRIPTION: SEQ ID NO: 4:

His Ser Thr Ser Gln Glu Gly Gln Asp Thr Ile His Gly His Arg Gly
1               5                   10                  15

Ser


(2) INFORMATION FOR SEQ ID NO: 5:

    (i) SEQUENCE CHARACTERISTICS:
        (A) LENGTH: 11 amino acids
        (B) TYPE: amino acid
        (C) STRANDEDNESS: single
        (D) TOPOLOGY: linear

    (ii) MOLECULE TYPE: peptide

    (iii) HYPOTHETICAL: NO

    (iv) ANTI-SENSE: NO




    (xi) SEQUENCE DESCRIPTION: SEQ ID NO: 5:

    Gly Gly Gln Gly Ser Arg His Gln Gln Ala Arg
    1               5                   10

(2) INFORMATION FOR SEQ ID NO: 6:

    (i) SEQUENCE CHARACTERISTICS:
        (A) LENGTH: 30 amino acids
        (B) TYPE: amino acid
        (C) STRANDEDNESS: single
        (D) TOPOLOGY: linear

    (ii) MOLECULE TYPE: peptide

    (iii) HYPOTHETICAL: NO

    (iv) ANTI-SENSE: NO




    (xi) SEQUENCE DESCRIPTION: SEQ ID NO: 6:

    Gln Gly Ser Arg His Gln Gln Ala Arg Asp Ser Ser Arg His Ser Thr
    1               5                   10                  15

    Ser Gln Glu Gly Gln Asp Thr Ile His Gly His Arg Gly Ser
                20                  25                  30

(2) INFORMATION FOR SEQ ID NO: 7:
```

(i) SEQUENCE CHARACTERISTICS:
        (A) LENGTH: 31 amino acids
        (B) TYPE: amino acid
        (C) STRANDEDNESS: single
        (D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 7:

Gln Gly Ser Arg His Gln Gln Ala Arg Asp Ser Ser Arg His Ser Ala
1               5                   10                  15

Ser Gln Asp Gly Gln Asp Thr Ile Arg Gly His Pro Gly Ser Ser
            20                  25                  30

(2) INFORMATION FOR SEQ ID NO: 8:

    (i) SEQUENCE CHARACTERISTICS:
        (A) LENGTH: 32 amino acids
        (B) TYPE: amino acid
        (C) STRANDEDNESS: single
        (D) TOPOLOGY: linear

    (ii) MOLECULE TYPE: peptide

    (iii) HYPOTHETICAL: NO

    (iv) ANTI-SENSE: NO

    (xi) SEQUENCE DESCRIPTION: SEQ ID NO: 8:

    His Ser Gly Ile Gly His Gly Gln Ala Ser Ser Ala Val Arg Asp Ser
    1               5                   10                  15

    Gly His Arg Gly Tyr Ser Gly Ser Gln Ala Ser Asp Asn Glu Gly His
                20                  25                  30

(2) INFORMATION FOR SEQ ID NO: 9:

    (i) SEQUENCE CHARACTERISTICS:
        (A) LENGTH: 12 amino acids
        (B) TYPE: amino acid
        (C) STRANDEDNESS: single
        (D) TOPOLOGY: linear

    (ii) MOLECULE TYPE: peptide

(iii) HYPOTHETICAL: NO

(iv) ANTI-SENSE: NO

(xi) SEQUENCE DESCRIPTION: SEQ ID NO: 9:

Arg Ala Gly His Gly His Ser Ala Asp Ser Ser Arg
1               5                   10

(2) INFORMATION FOR SEQ ID NO: 10:

   (i) SEQUENCE CHARACTERISTICS:
       (A) LENGTH: 10 amino acids
       (B) TYPE: amino acid
       (C) STRANDEDNESS: single
       (D) TOPOLOGY: linear

   (ii) MOLECULE TYPE: peptide

  (iii) HYPOTHETICAL: NO

   (iv) ANTI-SENSE: NO

   (ix) FEATURE:
       (A) NAME/KEY: Modified-site
       (B) LOCATION:6
       (D) OTHER INFORMATION:/product= "OTHER"
               /note= "Xaa represents citrulline"

   (xi) SEQUENCE DESCRIPTION: SEQ ID NO: 10:

Arg Gln Gly Ser Xaa His Gln Gln Ala Arg
1               5                   10

(2) INFORMATION FOR SEQ ID NO: 11:

   (i) SEQUENCE CHARACTERISTICS:
       (A) LENGTH: 14 amino acids
       (B) TYPE: amino acid
       (C) STRANDEDNESS: single
       (D) TOPOLOGY: linear

   (ii) MOLECULE TYPE: peptide'

  (iii) HYPOTHETICAL: NO

   (iv) ANTI-SENSE: NO

   (xi) SEQUENCE DESCRIPTION: SEQ ID NO: 11:

Arg His Gly Ser His His Gln Gln Ser Ala Asp Ser Ser Arg

```
                1              5                    10
```

(2) INFORMATION FOR SEQ ID NO: 12:

    (i) SEQUENCE CHARACTERISTICS:
        (A) LENGTH: 14 amino acids
        (B) TYPE: amino acid
        (C) STRANDEDNESS: single
        (D) TOPOLOGY: linear

    (ii) MOLECULE TYPE: peptide

    (iii) HYPOTHETICAL: NO

    (iv) ANTI-SENSE: NO

    (xi) SEQUENCE DESCRIPTION: SEQ ID NO: 12:

```
Arg His Ser Gln Val Gly Gln Gly Glu Ser Ser Gly Pro Arg
1               5                    10
```

(2) INFORMATION FOR SEQ ID NO: 13:

    (i) SEQUENCE CHARACTERISTICS:
        (A) LENGTH: 17 amino acids
        (B) TYPE: amino acid
        (C) STRANDEDNESS: single
        (D) TOPOLOGY: linear

    (ii) MOLECULE TYPE: peptide

    (iii) HYPOTHETICAL: NO

    (iv) ANTI-SENSE: NO

    (ix) FEATURE:
        (A) NAME/KEY: Modified-site
        (B) LOCATION:13
        (D) OTHER INFORMATION:/product= "OTHER"
            /note= "Xaa represents citrulline"

    (xi) SEQUENCE DESCRIPTION: SEQ ID NO: 13:

```
Arg His Ser Ala Ser Gln Asp Gly Gln Asp Thr Ile Xaa Gly His Pro
1               5                    10                   15

Gly
```

(2) INFORMATION FOR SEQ ID NO: 14:

    (i) SEQUENCE CHARACTERISTICS:
        (A) LENGTH: 15 amino acids
        (B) TYPE: amino acid

```
            (C) STRANDEDNESS: single
            (D) TOPOLOGY: linear

        (ii) MOLECULE TYPE: peptide

       (iii) HYPOTHETICAL: NO

        (iv) ANTI-SENSE: NO


        (ix) FEATURE:
            (A) NAME/KEY: Modified-site
            (B) LOCATION:13
            (D) OTHER INFORMATION:/product= "OTHER"
                   /note= "Xaa represents citrulline"


        (xi) SEQUENCE DESCRIPTION: SEQ ID NO: 14:

        Arg His Ser Ala Ser Gln Asp Gly Gln Asp Thr Ile Xaa Gly His
        1               5                  10                  15

    (2) INFORMATION FOR SEQ ID NO: 15:

        (i) SEQUENCE CHARACTERISTICS:
            (A) LENGTH: 15 amino acids
            (B) TYPE: amino acid
            (C) STRANDEDNESS: single
            (D) TOPOLOGY: linear

        (ii) MOLECULE TYPE: peptide

       (iii) HYPOTHETICAL: NO

        (iv) ANTI-SENSE: NO



        (xi) SEQUENCE DESCRIPTION: SEQ ID NO: 15:

        Arg His Ser Gly Ile Gly His Gly Gln Ala Ser Ser Ala Val Arg
        1               5                  10                  15

    (2) INFORMATION FOR SEQ ID NO: 16:

        (i) SEQUENCE CHARACTERISTICS:
            (A) LENGTH: 12 amino acids
            (B) TYPE: amino acid
            (C) STRANDEDNESS: single
            (D) TOPOLOGY: linear

        (ii) MOLECULE TYPE: peptide

       (iii) HYPOTHETICAL: NO

        (iv) ANTI-SENSE: NO
```

27

(xi) SEQUENCE DESCRIPTION: SEQ ID NO: 16:

Arg His Ser Ala Ser Gln Asp Gly Gln Asp Thr Ile
1               5                   10

(2) INFORMATION FOR SEQ ID NO: 17:

 (i) SEQUENCE CHARACTERISTICS:
  (A) LENGTH: 19 amino acids
  (B) TYPE: amino acid
  (C) STRANDEDNESS: single
  (D) TOPOLOGY: linear

 (ii) MOLECULE TYPE: peptide

 (iii) HYPOTHETICAL: NO

 (iv) ANTI-SENSE: NO

 (ix) FEATURE:
  (A) NAME/KEY: Modified-site
  (B) LOCATION:6
  (D) OTHER INFORMATION:/product= "OTHER"
   /note= "Xaa represents citrulline"

 (xi) SEQUENCE DESCRIPTION: SEQ ID NO: 17:

Arg Asp Ser Gly His Xaa Gly Tyr Ser Gly Ser Gln Ala Ser Asp Asn
1               5                   10                  15

Glu Gly His

(2) INFORMATION FOR SEQ ID NO: 18:

 (i) SEQUENCE CHARACTERISTICS:
  (A) LENGTH: 18 amino acids
  (B) TYPE: amino acid
  (C) STRANDEDNESS: single
  (D) TOPOLOGY: linear

 (ii) MOLECULE TYPE: peptide

 (iii) HYPOTHETICAL: NO

 (iv) ANTI-SENSE: NO

 (ix) FEATURE:
  (A) NAME/KEY: Modified-site
  (B) LOCATION:16
  (D) OTHER INFORMATION:/product= "OTHER"
   /note= "Xaa represents citrulline"

 (xi) SEQUENCE DESCRIPTION: SEQ ID NO: 18:

28

```
Arg His Ser Thr Ser Gln Glu Gly Gln Asp Thr Ile His Gly His Xaa
1               5                   10                  15

Gly Ser
```

(2) INFORMATION FOR SEQ ID NO: 19:

    (i) SEQUENCE CHARACTERISTICS:
        (A) LENGTH: 16 amino acids
        (B) TYPE: amino acid
        (C) STRANDEDNESS: single
        (D) TOPOLOGY: linear

    (ii) MOLECULE TYPE: peptide

    (iii) HYPOTHETICAL: NO

    (iv) ANTI-SENSE: NO

    (xi) SEQUENCE DESCRIPTION: SEQ ID NO: 19:

```
Arg Gly Tyr Ser Gly Ser Gln Ala Ser Asp Asn Glu Gly His Ser Glu
1               5                   10                  15
```

(2) INFORMATION FOR SEQ ID NO: 20:

    (i) SEQUENCE CHARACTERISTICS:
        (A) LENGTH: 11 amino acids
        (B) TYPE: amino acid
        (C) STRANDEDNESS: single
        (D) TOPOLOGY: linear

    (ii) MOLECULE TYPE: peptide

    (iii) HYPOTHETICAL: NO

    (iv) ANTI-SENSE: NO

    (xi) SEQUENCE DESCRIPTION: SEQ ID NO: 20:

```
Arg Asn Asp Glu Gln Ser Gly Asp Gly Ser Arg
1               5                   10
```

(2) INFORMATION FOR SEQ ID NO: 21:

    (i) SEQUENCE CHARACTERISTICS:
        (A) LENGTH: 18 amino acids
        (B) TYPE: amino acid
        (C) STRANDEDNESS: single
        (D) TOPOLOGY: linear

(ii) MOLECULE TYPE: peptide

(iii) HYPOTHETICAL: NO

(iv) ANTI-SENSE: NO

(ix) FEATURE:
    (A) NAME/KEY: Modified-site
    (B) LOCATION:12
    (D) OTHER INFORMATION:/product= "OTHER"
       /note= "Xaa represents citrulline"

(xi) SEQUENCE DESCRIPTION: SEQ ID NO: 21:

His Ser Ala Ser Gln Asp Gly Gln Asp Thr Ile Xaa Gly His Pro Gly
1          5                10              15

Ser Ser

(2) INFORMATION FOR SEQ ID NO: 22:

    (i) SEQUENCE CHARACTERISTICS:
        (A) LENGTH: 22 amino acids
        (B) TYPE: amino acid
        (C) STRANDEDNESS: single
        (D) TOPOLOGY: linear

    (ii) MOLECULE TYPE: peptide

    (iii) HYPOTHETICAL: NO

    (iv) ANTI-SENSE: NO

    (ix) FEATURE:
        (A) NAME/KEY: Modified-site
        (B) LOCATION:19
        (D) OTHER INFORMATION:/product= "OTHER"
           /note= "Xaa represents citrulline"

    (xi) SEQUENCE DESCRIPTION: SEQ ID NO: 22:

His Ser Gly Ile Gly His Gly Gln Ala Ser Ser Ala Val Arg Asp Ser
1          5                10              15

Gly His Xaa Gly Tyr Ser
          20

(2) INFORMATION FOR SEQ ID NO: 23:

    (i) SEQUENCE CHARACTERISTICS:
        (A) LENGTH: 18 amino acids
        (B) TYPE: amino acid
        (C) STRANDEDNESS: single
        (D) TOPOLOGY: linear

```
       (ii) MOLECULE TYPE: peptide

      (iii) HYPOTHETICAL: NO

       (iv) ANTI-SENSE: NO


       (ix) FEATURE:
            (A) NAME/KEY: Modified-site
            (B) LOCATION:5
            (D) OTHER INFORMATION:/product= "OTHER"
                 /note= "Xaa represents citrulline"


       (xi) SEQUENCE DESCRIPTION: SEQ ID NO: 23:

       Asp Ser Gly His Xaa Gly Tyr Ser Gly Ser Gln Ala Ser Asp Asn Glu
       1               5                   10                  15

       Gly His


 (2) INFORMATION FOR SEQ ID NO: 24:

       (i) SEQUENCE CHARACTERISTICS:
            (A) LENGTH: 17 amino acids
            (B) TYPE: amino acid
            (C) STRANDEDNESS: single
            (D) TOPOLOGY: linear

       (ii) MOLECULE TYPE: peptide

      (iii) HYPOTHETICAL: NO

       (iv) ANTI-SENSE: NO


       (ix) FEATURE:
            (A) NAME/KEY: Modified-site
            (B) LOCATION:15
            (D) OTHER INFORMATION:/product= "OTHER"
                 /note= "Xaa represents citrulline"


       (xi) SEQUENCE DESCRIPTION: SEQ ID NO: 24:

       His Ser Thr Ser Gln Glu Gly Gln Asp Thr Ile His Gly His Xaa Gly
       1               5                   10                  15

       Ser


 (2) INFORMATION FOR SEQ ID NO: 25:

       (i) SEQUENCE CHARACTERISTICS:
            (A) LENGTH: 11 amino acids
            (B) TYPE: amino acid
            (C) STRANDEDNESS: single
            (D) TOPOLOGY: linear
```

```
(ii) MOLECULE TYPE: peptide

(iii) HYPOTHETICAL: NO

(iv) ANTI-SENSE: NO


(ix) FEATURE:
     (A) NAME/KEY: Modified-site
     (B) LOCATION:6
     (D) OTHER INFORMATION:/product= "OTHER"
             /note= "Xaa represents citrulline"


(xi) SEQUENCE DESCRIPTION: SEQ ID NO: 25:

Gly Gly Gln Gly Ser Xaa His Gln Gln Ala Arg
1               5                   10
```

(2) INFORMATION FOR SEQ ID NO: 26:

```
    (i) SEQUENCE CHARACTERISTICS:
        (A) LENGTH: 13 amino acids
        (B) TYPE: amino acid
        (C) STRANDEDNESS: single
        (D) TOPOLOGY: linear

   (ii) MOLECULE TYPE: peptide

  (iii) HYPOTHETICAL: NO

   (iv) ANTI-SENSE: NO



   (xi) SEQUENCE DESCRIPTION: SEQ ID NO: 26:

   Gly Gly Ala Gly His Gly His Ser Ala Asp Ser Ser Arg
   1               5                   10
```

(2) INFORMATION FOR SEQ ID NO: 27:

```
    (i) SEQUENCE CHARACTERISTICS:
        (A) LENGTH: 15 amino acids
        (B) TYPE: amino acid
        (C) STRANDEDNESS: single
        (D) TOPOLOGY: linear

   (ii) MOLECULE TYPE: peptide

  (iii) HYPOTHETICAL: NO

   (iv) ANTI-SENSE: NO



   (xi) SEQUENCE DESCRIPTION: SEQ ID NO: 27:
```

```
Gly Gly His Gly Ser His His Gln Gln Ser Ala Asp Ser Ser Arg
1               5                   10                  15
```

(2) INFORMATION FOR SEQ ID NO: 28:

    (i) SEQUENCE CHARACTERISTICS:
        (A) LENGTH: 16 amino acids
        (B) TYPE: amino acid
        (C) STRANDEDNESS: single
        (D) TOPOLOGY: linear

    (ii) MOLECULE TYPE: peptide

    (iii) HYPOTHETICAL: NO

    (iv) ANTI-SENSE: NO

    (xi) SEQUENCE DESCRIPTION: SEQ ID NO: 28:

```
Gly Gly Asn Asp Glu Gln Ser Gly Asp Gly Ser Arg His Ser Gly Ser
1               5                   10                  15
```

(2) INFORMATION FOR SEQ ID NO: 29:

    (i) SEQUENCE CHARACTERISTICS:
        (A) LENGTH: 15 amino acids
        (B) TYPE: amino acid
        (C) STRANDEDNESS: single
        (D) TOPOLOGY: linear

    (ii) MOLECULE TYPE: peptide

    (iii) HYPOTHETICAL: NO

    (iv) ANTI-SENSE: NO

    (xi) SEQUENCE DESCRIPTION: SEQ ID NO: 29:

```
Ser Arg His Ser Gln Val Gly Gln Gly Glu Ser Ser Gly Pro Arg
1               5                   10                  15
```

(2) INFORMATION FOR SEQ ID NO: 30:

    (i) SEQUENCE CHARACTERISTICS:
        (A) LENGTH: 36 base pairs
        (B) TYPE: nucleic acid
        (C) STRANDEDNESS: single
        (D) TOPOLOGY: linear

    (ii) MOLECULE TYPE: DNA (genomic)

    (iii) HYPOTHETICAL: NO

(iv) ANTI-SENSE: NO

(xi) SEQUENCE DESCRIPTION: SEQ ID NO: 30:

GAATTCGCCA CCATGGGGTC TTTCCTCTAC CAGGTG          36

(2) INFORMATION FOR SEQ ID NO: 31:

    (i) SEQUENCE CHARACTERISTICS:
        (A) LENGTH: 8 amino acids
        (B) TYPE: amino acid
        (C) STRANDEDNESS: single
        (D) TOPOLOGY: linear

    (ii) MOLECULE TYPE: peptide

    (iii) HYPOTHETICAL: NO

    (iv) ANTI-SENSE: NO

    (xi) SEQUENCE DESCRIPTION: SEQ ID NO: 31:

    Met Gly Ser Phe Leu Tyr Gln Val
    1               5

(2) INFORMATION FOR SEQ ID NO: 32:

    (i) SEQUENCE CHARACTERISTICS:
        (A) LENGTH: 36 base pairs
        (B) TYPE: nucleic acid
        (C) STRANDEDNESS: single
        (D) TOPOLOGY: linear

    (ii) MOLECULE TYPE: DNA (genomic)

    (iii) HYPOTHETICAL: NO

    (iv) ANTI-SENSE: NO

    (xi) SEQUENCE DESCRIPTION: SEQ ID NO: 32:

TGCAGACCTG TAAGTCCTAG AGGGCCCGAG ATCTGG          36

(2) INFORMATION FOR SEQ ID NO: 33:

    (i) SEQUENCE CHARACTERISTICS:
        (A) LENGTH: 10 amino acids
        (B) TYPE: amino acid
        (C) STRANDEDNESS: single
        (D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 33:

Thr Ser Gly His Ser Gly Ser Pro Gly His
1               5                   10

(2) INFORMATION FOR SEQ ID NO: 34:

    (i) SEQUENCE CHARACTERISTICS:
        (A) LENGTH: 18 amino acids
        (B) TYPE: amino acid
        (C) STRANDEDNESS: single
        (D) TOPOLOGY: linear

    (ii) MOLECULE TYPE: peptide

    (iii) HYPOTHETICAL: NO

    (iv) ANTI-SENSE: NO

    (ix) FEATURE:
        (A) NAME/KEY: Modified-site
        (B) LOCATION:12
        (D) OTHER INFORMATION:/product= "OTHER"
            /note= "Xaa represents a citrulline residue"

    (xi) SEQUENCE DESCRIPTION: SEQ ID NO: 34:

His Ser Ala Ser Gln Asp Gly Gln Asp Thr Ile Xaa Gly His Pro Gly
1               5                   10                  15

Ser Ser

(2) INFORMATION FOR SEQ ID NO: 35:

    (i) SEQUENCE CHARACTERISTICS:
        (A) LENGTH: 22 amino acids
        (B) TYPE: amino acid
        (C) STRANDEDNESS: single
        (D) TOPOLOGY: linear

    (ii) MOLECULE TYPE: peptide

    (iii) HYPOTHETICAL: NO

    (iv) ANTI-SENSE: NO

```
(ix) FEATURE:
    (A) NAME/KEY: Modified-site
    (B) LOCATION:19
    (D) OTHER INFORMATION:/product= "OTHER"
        /note= "Xaa represents a citrulline residue"


(xi) SEQUENCE DESCRIPTION: SEQ ID NO: 35:

His Ser Gly Ile Gly His Gly Gln Ala Ser Ser Ala Val Arg Asp Ser
1               5                   10                  15

Gly His Xaa Gly Tyr Ser
            20
```

(2) INFORMATION FOR SEQ ID NO: 36:

```
    (i) SEQUENCE CHARACTERISTICS:
        (A) LENGTH: 18 amino acids
        (B) TYPE: amino acid
        (C) STRANDEDNESS: single
        (D) TOPOLOGY: linear

    (ii) MOLECULE TYPE: peptide

    (iii) HYPOTHETICAL: NO

    (iv) ANTI-SENSE: NO


    (ix) FEATURE:
        (A) NAME/KEY: Modified-site
        (B) LOCATION:5
        (D) OTHER INFORMATION:/product= "OTHER"
            /note= "Xaa represents a citrulline residue"


    (xi) SEQUENCE DESCRIPTION: SEQ ID NO: 36:

Asp Ser Gly His Xaa Gly Tyr Ser Gly Ser Gln Ala Ser Asp Asn Glu
1               5                   10                  15

Gly His
```

(2) INFORMATION FOR SEQ ID NO: 37:

```
    (i) SEQUENCE CHARACTERISTICS:
        (A) LENGTH: 17 amino acids
        (B) TYPE: amino acid
        (C) STRANDEDNESS: single
        (D) TOPOLOGY: linear

    (ii) MOLECULE TYPE: peptide

    (iii) HYPOTHETICAL: NO

    (iv) ANTI-SENSE: NO
```

```
(ix) FEATURE:
      (A) NAME/KEY: Modified-site
      (B) LOCATION:15
      (D) OTHER INFORMATION:/product= "OTHER"
            /note= "Xaa represents a citrulline residue"


(xi) SEQUENCE DESCRIPTION: SEQ ID NO: 37:

His Ser Thr Ser Gln Glu Gly Gln Asp Thr Ile His Gly His Xaa Gly
1               5                   10                  15

Ser


(2) INFORMATION FOR SEQ ID NO: 38:

    (i) SEQUENCE CHARACTERISTICS:
        (A) LENGTH: 11 amino acids
        (B) TYPE: amino acid
        (C) STRANDEDNESS: single
        (D) TOPOLOGY: linear

    (ii) MOLECULE TYPE: peptide

    (iii) HYPOTHETICAL: NO

    (iv) ANTI-SENSE: NO


    (ix) FEATURE:
        (A) NAME/KEY: Modified-site
        (B) LOCATION:6
        (D) OTHER INFORMATION:/product= "OTHER"
              /note= "Xaa represents a citrulline residue"


    (xi) SEQUENCE DESCRIPTION: SEQ ID NO: 38:

    Gly Gly Gln Gly Ser Xaa His Gln Gln Ala Arg
    1               5                   10

(2) INFORMATION FOR SEQ ID NO: 39:

    (i) SEQUENCE CHARACTERISTICS:
        (A) LENGTH: 30 amino acids
        (B) TYPE: amino acid
        (C) STRANDEDNESS: single
        (D) TOPOLOGY: linear

    (ii) MOLECULE TYPE: peptide

    (iii) HYPOTHETICAL: NO

    (iv) ANTI-SENSE: NO


    (ix) FEATURE:
        (A) NAME/KEY: Modified-site
```

```
        (B) LOCATION:4
        (D) OTHER INFORMATION:/product= "OTHER"
             /note= "Xaa represents a citrulline residue"


    (ix) FEATURE:
        (A) NAME/KEY: Modified-site
        (B) LOCATION:28
        (D) OTHER INFORMATION:/product= "OTHER"
             /note= "Xaa represents a citrulline residue"



    (xi) SEQUENCE DESCRIPTION: SEQ ID NO: 39:

    Gln Gly Ser Xaa His Gln Gln Ala Arg Asp Ser Ser Arg His Ser Thr
    1               5                   10                  15

    Ser Gln Glu Gly Gln Asp Thr Ile His Gly His Xaa Gly Ser
                20                  25                  30


(2) INFORMATION FOR SEQ ID NO: 40:

    (i) SEQUENCE CHARACTERISTICS:
        (A) LENGTH: 31 amino acids
        (B) TYPE: amino acid
        (C) STRANDEDNESS: single
        (D) TOPOLOGY: linear

    (ii) MOLECULE TYPE: peptide

    (iii) HYPOTHETICAL: NO

    (iv) ANTI-SENSE: NO


    (ix) FEATURE:
        (A) NAME/KEY: Modified-site
        (B) LOCATION:4
        (D) OTHER INFORMATION:/product= "OTHER"
             /note= "Xaa represents a citrulline residue"

    (ix) FEATURE:
        (A) NAME/KEY: Modified-site
        (B) LOCATION:25
        (D) OTHER INFORMATION:/product= "OTHER"
             /note= "Xaa represents a citrulline residue"


    (xi) SEQUENCE DESCRIPTION: SEQ ID NO: 40:

    Gln Gly Ser Xaa His Gln Gln Ala Arg Asp Ser Ser Arg His Ser Ala
    1               5                   10                  15

    Ser Gln Asp Gly Gln Asp Thr Ile Xaa Gly His Pro Gly Ser Ser
                20                  25                  30

(2) INFORMATION FOR SEQ ID NO: 41:

    (i) SEQUENCE CHARACTERISTICS:
        (A) LENGTH: 32 amino acids
```

```
            (B)  TYPE: amino acid
            (C)  STRANDEDNESS: single
            (D)  TOPOLOGY: linear

       (ii)  MOLECULE TYPE: peptide

      (iii)  HYPOTHETICAL: NO


       (ix)  FEATURE:
            (A)  NAME/KEY: Modified-site
            (B)  LOCATION:19
            (D)  OTHER INFORMATION:/product= "OTHER"
                 /note= "Xaa represents a citrulline residue"


       (xi)  SEQUENCE DESCRIPTION: SEQ ID NO: 41:

       His Ser Gly Ile Gly His Gly Gln Ala Ser Ser Ala Val Arg Asp Ser
       1               5                   10                  15


       Gly His Xaa Gly Tyr Ser Gly Ser Gln Ala Ser Asp Asn Glu Gly His
                   20                  25                  30



 (2) INFORMATION FOR SEQ ID NO: 42:

       (i)  SEQUENCE CHARACTERISTICS:
            (A)  LENGTH: 1006 base pairs
            (B)  TYPE: nucleic acid
            (C)  STRANDEDNESS: single
            (D)  TOPOLOGY: linear

      (ii)  MOLECULE TYPE: DNA (genomic)

     (iii)  HYPOTHETICAL: NO

      (iv)  ANTI-SENSE: NO


       (ix)  FEATURE:
            (A)  NAME/KEY: CDS
            (B)  LOCATION:13..1006

       (ix)  FEATURE:
            (A)  NAME/KEY: mat_peptide
            (B)  LOCATION:13..1003


       (xi)  SEQUENCE DESCRIPTION: SEQ ID NO: 42:

GAATTCGCCA CC ATG GGG TCT TTC CTC TAC CAG GTG AGC ACT CAT GAA          48
              Met Gly Ser Phe Leu Tyr Gln Val Ser Thr His Glu
              1               5                   10

CAG TCT GAG TCC GCC CAT GGA CGG ACC GGG ACC AGC ACT GGA GGA AGA        96
Gln Ser Glu Ser Ala His Gly Arg Thr Gly Thr Ser Thr Gly Gly Arg
        15                  20                  25
```

```
CAA GGA TCC CAC CAC GAG CAG GCA CGA GAC AGC TCC AGG CAC TCA ACG       144
Gln Gly Ser His His Glu Gln Ala Arg Asp Ser Ser Arg His Ser Thr
     30                  35                  40

TCC CAA GAG GGT CAG GAC ACC ATT CAT GGA CAC CGG GGG TCA AGC AGT       192
Ser Gln Glu Gly Gln Asp Thr Ile His Gly His Arg Gly Ser Ser Ser
 45                  50                  55                  60

GGA GGA AGG CAG GGA TCC CAC TAC GAG CAA TCG GTA GAT AGA TCT GGA       240
Gly Gly Arg Gln Gly Ser His Tyr Glu Gln Ser Val Asp Arg Ser Gly
                 65                  70                  75

CAC TCA GGG TCT CAT CAC AGC CAC ACC ACA TCC CAG GGA AGG TCT GAT       288
His Ser Gly Ser His His Ser His Thr Thr Ser Gln Gly Arg Ser Asp
                 80                  85                  90

GCC TCC CAT GGG CAG TCA GGA TCC AGA AGT GCA AGC AGA CAA ACT CGT       336
Ala Ser His Gly Gln Ser Gly Ser Arg Ser Ala Ser Arg Gln Thr Arg
             95                  100                 105

AAC GAT GAA CAA TCA GGA GAC GGC TCC AGG CAC TCA GGG TCG CGT CAC       384
Asn Asp Glu Gln Ser Gly Asp Gly Ser Arg His Ser Gly Ser Arg His
     110                 115                 120

CAT GAA GCT TCC TCT CGG GCC GAC AGC TCT AGA CAC TCG CAG GTG GGC       432
His Glu Ala Ser Ser Arg Ala Asp Ser Ser Arg His Ser Gln Val Gly
125                 130                 135                 140

CAG GGA CAA TCA GAG GGG CCC AGG ACA AGC AGG AAC TGG GGA TCC AGT       480
Gln Gly Gln Ser Glu Gly Pro Arg Thr Ser Arg Asn Trp Gly Ser Ser
                 145                 150                 155

TTT AGC CAG GAC AGT GAC AGT GAG GGA CAT TCA GAA GAC TCT GAG AGG       528
Phe Ser Gln Asp Ser Asp Ser Glu Gly His Ser Glu Asp Ser Glu Arg
                 160                 165                 170

TGG TCT GGG TCT GCT TCC AGA AAC CAT CAT GGA TCT GCT CAG GAG CAG       576
Trp Ser Gly Ser Ala Ser Arg Asn His His Gly Ser Ala Gln Glu Gln
                 175                 180                 185

CTA AGA GAT GGC TCC AGA CAC CCC AGG TCC CAT CAA GAA GAC AGA GCT       624
Leu Arg Asp Gly Ser Arg His Pro Arg Ser His Gln Glu Asp Arg Ala
     190                 195                 200

GGT CAT GGG CAC TCT GCA GAC AGC TCC AGA CAA TCA GGC ACT CGT CAC       672
Gly His Gly His Ser Ala Asp Ser Ser Arg Gln Ser Gly Thr Arg His
205                 210                 215                 220

ACA CAG ACT TCC TCT GGT GGA CAG GCT GCA TCA TCC CAT GAA CAG GCA       720
Thr Gln Thr Ser Ser Gly Gly Gln Ala Ala Ser Ser His Glu Gln Ala
                 225                 230                 235

AGA TCA AGT GCA GGA GAA AGA CAT GGA TCC CAC CAC CAG CAG TCA GCA       768
Arg Ser Ser Ala Gly Glu Arg His Gly Ser His His Gln Gln Ser Ala
                 240                 245                 250

GAC AGC TCC AGA CAC TCA GGC ATT GGG CAC GGA CAA GCT TCA TCT GCA       816
Asp Ser Ser Arg His Ser Gly Ile Gly His Gly Gln Ala Ser Ser Ala
                 255                 260                 265
```

```
GTC AGA GAC AGT GGA CAC CGA GGG TAC AGT GGT AGT CAG GCC AGT GAC        864
Val Arg Asp Ser Gly His Arg Gly Tyr Ser Gly Ser Gln Ala Ser Asp
    270                 275                 280

AAT GAG GGA CAT TCA GAA GAC TCA GAC ACA CAG TCA GTG TCA GCC CAC        912
Asn Glu Gly His Ser Glu Asp Ser Asp Thr Gln Ser Val Ser Ala His
285                 290                 295                 300

GGA CAG GCT GGG TCC CAT CAG CAG TGC CAC CAA GAG TCC GCA CGT GGC        960
Gly Gln Ala Gly Ser His Gln Gln Cys His Gln Glu Ser Ala Arg Gly
                305                 310                 315

CGG TCA GGG GAA ACG TCT GGA CAT TCA GGA TCT CCC GGG CTC TAG  A        1006
Arg Ser Gly Glu Thr Ser Gly His Ser Gly Ser Pro Gly Leu  *
                320                 325                 330
```

(2) INFORMATION FOR SEQ ID NO: 43:

    (i) SEQUENCE CHARACTERISTICS:
      (A) LENGTH: 331 amino acids
      (B) TYPE: amino acid
      (D) TOPOLOGY: linear

    (ii) MOLECULE TYPE: protein
    (xi) SEQUENCE DESCRIPTION: SEQ ID NO: 43:

```
Met Gly Ser Phe Leu Tyr Gln Val Ser Thr His Glu Gln Ser Glu Ser
  1               5                   10                  15

Ala His Gly Arg Thr Gly Thr Ser Thr Gly Gly Arg Gln Gly Ser His
              20                  25                  30

His Glu Gln Ala Arg Asp Ser Ser Arg His Ser Thr Ser Gln Glu Gly
          35                  40                  45

Gln Asp Thr Ile His Gly His Arg Gly Ser Ser Ser Gly Gly Arg Gln
          50                  55                  60

Gly Ser His Tyr Glu Gln Ser Val Asp Arg Ser Gly His Ser Gly Ser
 65                 70                  75                  80

His His Ser His Thr Thr Ser Gln Gly Arg Ser Asp Ala Ser His Gly
              85                  90                  95

Gln Ser Gly Ser Arg Ser Ala Ser Arg Gln Thr Arg Asn Asp Glu Gln
          100                 105                 110

Ser Gly Asp Gly Ser Arg His Ser Gly Ser Arg His His Glu Ala Ser
          115                 120                 125

Ser Arg Ala Asp Ser Ser Arg His Ser Gln Val Gly Gln Gly Gln Ser
          130                 135                 140

Glu Gly Pro Arg Thr Ser Arg Asn Trp Gly Ser Ser Phe Ser Gln Asp
145                 150                 155                 160

Ser Asp Ser Glu Gly His Ser Glu Asp Ser Glu Arg Trp Ser Gly Ser
              165                 170                 175
```

```
Ala Ser Arg Asn His His Gly Ser Ala Gln Glu Gln Leu Arg Asp Gly
            180                 185                 190

Ser Arg His Pro Arg Ser His Gln Glu Asp Arg Ala Gly His Gly His
            195                 200                 205

Ser Ala Asp Ser Ser Arg Gln Ser Gly Thr Arg His Thr Gln Thr Ser
            210                 215                 220

Ser Gly Gly Gln Ala Ala Ser Ser His Glu Gln Ala Arg Ser Ser Ala
225                 230                 235                 240

Gly Glu Arg His Gly Ser His His Gln Gln Ser Ala Asp Ser Ser Arg
            245                 250                 255

His Ser Gly Ile Gly His Gly Gln Ala Ser Ser Ala Val Arg Asp Ser
            260                 265                 270

Gly His Arg Gly Tyr Ser Gly Ser Gln Ala Ser Asp Asn Glu Gly His
            275                 280                 285

Ser Glu Asp Ser Asp Thr Gln Ser Val Ser Ala His Gly Gln Ala Gly
            290                 295                 300

Ser His Gln Gln Cys His Gln Glu Ser Ala Arg Gly Arg Ser Gly Glu
305                 310                 315                 320

Thr Ser Gly His Ser Gly Ser Pro Gly Leu  *
            325                 330
```

(2) INFORMATION FOR SEQ ID NO: 44:

      (i) SEQUENCE CHARACTERISTICS:
          (A) LENGTH: 1006 base pairs
          (B) TYPE: nucleic acid
          (C) STRANDEDNESS: single
          (D) TOPOLOGY: linear

     (ii) MOLECULE TYPE: DNA (genomic)

    (iii) HYPOTHETICAL: NO

    (iv) ANTI-SENSE: NO

     (ix) FEATURE:
          (A) NAME/KEY: CDS
          (B) LOCATION:13..1006

     (ix) FEATURE:
          (A) NAME/KEY: mat_peptide
          (B) LOCATION:13..1003

     (xi) SEQUENCE DESCRIPTION: SEQ ID NO: 44:

```
GAATTCGCCA CC ATG GGG TCT TTC CTC TAC CAG GTG AGC ACT CAT GAA        48
              Met Gly Ser Phe Leu Tyr Gln Val Ser Thr His Glu
                1               5                   10
```

```
CAG TCT GAG TCC GCC CAT GGA CGG ACC GGG ACC AGC ACT GGA GGA AGA        96
Gln Ser Glu Ser Ala His Gly Arg Thr Gly Thr Ser Thr Gly Gly Arg
        15                  20                  25

CAA GGA TCC CAC CAC GAG CAG GCA CGA GAC AGC TCC AGG CAC TCA ACG        144
Gln Gly Ser His His Glu Gln Ala Arg Asp Ser Ser Arg His Ser Thr
    30                  35                  40

TCC CAA GAG GGT CAG GAC ACC ATT CAT GGA CAC CGG GGG TCA AGC AGT        192
Ser Gln Glu Gly Gln Asp Thr Ile His Gly His Arg Gly Ser Ser Ser
45                  50                  55                  60

GGA GGA AGG CAG GGA TCC CAC TAC GAG CAA TCG GTA GAT AGA TCT GGA        240
Gly Gly Arg Gln Gly Ser His Tyr Glu Gln Ser Val Asp Arg Ser Gly
                65                  70                  75

CAC TCA GGG TCT CAT CAC AGC CAC ACC ACA TCC CAG GGA AGG TCT GAT        288
His Ser Gly Ser His His Ser His Thr Thr Ser Gln Gly Arg Ser Asp
            80                  85                  90

GCC TCC CAT GGG CAG TCA GGA TCC AGA AGT GCA AGC AGA CAA ACT CGT        336
Ala Ser His Gly Gln Ser Gly Ser Arg Ser Ala Ser Arg Gln Thr Arg
        95                  100                 105

AAC GAT GAA CAA TCA GGA GAC GGC TCC AGG CAC TCA GGG TCG CGC CAC        384
Asn Asp Glu Gln Ser Gly Asp Gly Ser Arg His Ser Gly Ser Arg His
    110                 115                 120

CAT GAA GCT TCC TCT CGG GCC GAC AGC TCT AGA CAC TCG CAG GTG GGC        432
His Glu Ala Ser Ser Arg Ala Asp Ser Ser Arg His Ser Gln Val Gly
125                 130                 135                 140

CAG GGA CAA TCA GAG GGG CCC AGG ACA AGC AGG AAC TGG GGA TCC AGT        480
Gln Gly Gln Ser Glu Gly Pro Arg Thr Ser Arg Asn Trp Gly Ser Ser
            145                 150                 155

TTT AGC CAG GAC AGT GAC AGT GAG GGA CAT TCA GAA GAC TCT GAG AGG        528
Phe Ser Gln Asp Ser Asp Ser Glu Gly His Ser Glu Asp Ser Glu Arg
            160                 165                 170

TGG TCT GGG TCT GCT TCC AGA AAC CAT CAT GGA TCT GCT CAG GAG CAG        576
Trp Ser Gly Ser Ala Ser Arg Asn His His Gly Ser Ala Gln Glu Gln
        175                 180                 185

CTA AGA GAT GGC TCC AGA CAC CCC AGG TCC CAT CAA GAA GGC AGA GCT        624
Leu Arg Asp Gly Ser Arg His Pro Arg Ser His Gln Glu Gly Arg Ala
        190                 195                 200

GGT CAT GGG CAC TCT GCA GAC AGC TCC AGA CAA TCA GGC ACT CGT CAC        672
Gly His Gly His Ser Ala Asp Ser Ser Arg Gln Ser Gly Thr Arg His
205                 210                 215                 220

ACA CAG ACT TCC TCT GGT GGA CAG GCT GCA TCA TCC CAT GAA CGG GCA        720
Thr Gln Thr Ser Ser Gly Gly Gln Ala Ala Ser Ser His Glu Arg Ala
            225                 230                 235

AGA TCA AGT GCA GGA GAA AGA CAT GGA TCC CAC CAC CAG CAG TCA GCA        768
Arg Ser Ser Ala Gly Glu Arg His Gly Ser His His Gln Gln Ser Ala
            240                 245                 250
```

```
GAC AGC TCC AGA CAC TCA GGC ATT GGG CAC GGA CAA GCT TCA TCT GCA        816
Asp Ser Ser Arg His Ser Gly Ile Gly His Gly Gln Ala Ser Ser Ala
        255                 260                 265

GTC AGA GAC AGT GGA CAC CGA GGG TAC AGT GGT AGT CAG GCC AGT GAC        864
Val Arg Asp Ser Gly His Arg Gly Tyr Ser Gly Ser Gln Ala Ser Asp
        270                 275                 280

AAT GAG GGA CAT TCA GAA GAC TCA GAC ACA CAG TCA GTG TCA GCC CAC        912
Asn Glu Gly His Ser Glu Asp Ser Asp Thr Gln Ser Val Ser Ala His
285                 290                 295                 300

GGA CAG GCT GGG TCC CAT CAG CAG AGC CAC CAA GAG TCC GCA CGT GGC        960
Gly Gln Ala Gly Ser His Gln Gln Ser His Gln Glu Ser Ala Arg Gly
            305                 310                 315

CGG TCA GGG GAA ACG TCT GGA CAT TCA GGA TCT CCC GGG CTC TAG  A        1006
Arg Ser Gly Glu Thr Ser Gly His Ser Gly Ser Pro Gly Leu  *
            320                 325                 330


(2) INFORMATION FOR SEQ ID NO: 45:

        (i) SEQUENCE CHARACTERISTICS:
            (A) LENGTH: 331 amino acids
            (B) TYPE: amino acid
            (D) TOPOLOGY: linear

        (ii) MOLECULE TYPE: protein
        (xi) SEQUENCE DESCRIPTION: SEQ ID NO: 45:

Met Gly Ser Phe Leu Tyr Gln Val Ser Thr His Glu Gln Ser Glu Ser
 1               5                  10                  15

Ala His Gly Arg Thr Gly Thr Ser Thr Gly Gly Arg Gln Gly Ser His
            20                  25                  30

His Glu Gln Ala Arg Asp Ser Ser Arg His Ser Thr Ser Gln Glu Gly
        35                  40                  45

Gln Asp Thr Ile His Gly His Arg Gly Ser Ser Ser Gly Gly Arg Gln
        50                  55                  60

Gly Ser His Tyr Glu Gln Ser Val Asp Arg Ser Gly His Ser Gly Ser
 65                 70                  75                  80

His His Ser His Thr Thr Ser Gln Gly Arg Ser Asp Ala Ser His Gly
                85                  90                  95

Gln Ser Gly Ser Arg Ser Ala Ser Arg Gln Thr Arg Asn Asp Glu Gln
            100                 105                 110

Ser Gly Asp Gly Ser Arg His Ser Gly Ser Arg His His Glu Ala Ser
        115                 120                 125

Ser Arg Ala Asp Ser Ser Arg His Ser Gln Val Gly Gln Gly Gln Ser
        130                 135                 140

Glu Gly Pro Arg Thr Ser Arg Asn Trp Gly Ser Ser Phe Ser Gln Asp
```

```
              145              150              155              160

Ser Asp Ser Glu Gly His Ser Glu Asp Ser Glu Arg Trp Ser Gly Ser
                165              170              175

Ala Ser Arg Asn His His Gly Ser Ala Gln Glu Gln Leu Arg Asp Gly
            180              185              190

Ser Arg His Pro Arg Ser His Gln Glu Gly Arg Ala Gly His Gly His
            195              200              205

Ser Ala Asp Ser Ser Arg Gln Ser Gly Thr Arg His Thr Gln Thr Ser
        210              215              220

Ser Gly Gly Gln Ala Ala Ser Ser His Glu Arg Ala Arg Ser Ser Ala
225              230              235              240

Gly Glu Arg His Gly Ser His His Gln Gln Ser Ala Asp Ser Ser Arg
                245              250              255

His Ser Gly Ile Gly His Gly Gln Ala Ser Ser Ala Val Arg Asp Ser
            260              265              270

Gly His Arg Gly Tyr Ser Gly Ser Gln Ala Ser Asp Asn Glu Gly His
            275              280              285

Ser Glu Asp Ser Asp Thr Gln Ser Val Ser Ala His Gly Gln Ala Gly
        290              295              300

Ser His Gln Gln Ser His Gln Glu Ser Ala Arg Gly Arg Ser Gly Glu
305              310              315              320

Thr Ser Gly His Ser Gly Ser Pro Gly Leu  *
            325              330
```

(2) INFORMATION FOR SEQ ID NO: 46:

    (i) SEQUENCE CHARACTERISTICS:
        (A) LENGTH: 1006 base pairs
        (B) TYPE: nucleic acid
        (C) STRANDEDNESS: single
        (D) TOPOLOGY: linear

    (ii) MOLECULE TYPE: DNA (genomic)

    (iii) HYPOTHETICAL: NO

    (iv) ANTI-SENSE: NO

    (ix) FEATURE:
        (A) NAME/KEY: CDS
        (B) LOCATION:13..1006

    (ix) FEATURE:
        (A) NAME/KEY: mat_peptide
        (B) LOCATION:13..1003

(xi) SEQUENCE DESCRIPTION: SEQ ID NO: 46:

```
GAATTCGCCA CC ATG GGG TCT TTC CTC TAC CAG GTG GGC ACT CAT GAA                48
              Met Gly Ser Phe Leu Tyr Gln Val Gly Thr His Glu
               1               5                   10

CAG TCT GAG TCC GCC CAT GGA CGG ACC GGG ACC AGC ACT GGA GGA AGA                96
Gln Ser Glu Ser Ala His Gly Arg Thr Gly Thr Ser Thr Gly Gly Arg
         15                  20                  25

CAA GGA TCC CAC CAC GAG CAG GCA CGA GAC AGC TCC AGG CAC TCA ACG               144
Gln Gly Ser His His Glu Gln Ala Arg Asp Ser Ser Arg His Ser Thr
         30                  35                  40

TCC CAA GAG GGT CAG GAC ACC ATT CAT GGA CAC CGG GGG TCA AGC AGT               192
Ser Gln Glu Gly Gln Asp Thr Ile His Gly His Arg Gly Ser Ser Ser
 45                  50                  55                  60

GGA GGA AGG CAG GGA TCC CAC TAC GAG CAA TCG GTA GAT AGA TCT GGA               240
Gly Gly Arg Gln Gly Ser His Tyr Glu Gln Ser Val Asp Arg Ser Gly
                 65                  70                  75

CAC TCA GGG TCT CAT CAC AGC CAC ACC ACA TCC CAG GGA AGG TCT GAT               288
His Ser Gly Ser His His Ser His Thr Thr Ser Gln Gly Arg Ser Asp
                 80                  85                  90

GCC TCC CAT GGG CAG TCA GGA TCC AGA AGT GCA AGC AGA CAA ACT CGT               336
Ala Ser His Gly Gln Ser Gly Ser Arg Ser Ala Ser Arg Gln Thr Arg
                 95                  100                 105

AAC GAT GAA CAA TCA GGA GAC GGC TCC AGG CAC TCA GGG TCG CGT CAC               384
Asn Asp Glu Gln Ser Gly Asp Gly Ser Arg His Ser Gly Ser Arg His
         110                 115                 120

CAT GAA GCT TCC TCT CGG GCC GAC AGC TCT AGA CAC TCA CAG GCA GTC               432
His Glu Ala Ser Ser Arg Ala Asp Ser Ser Arg His Ser Gln Ala Val
125                 130                 135                 140

CAG GGA CAA TCA GAG GGG CCC AGG ACA AGC AGG AAC TGG GGA TCC AGT               480
Gln Gly Gln Ser Glu Gly Pro Arg Thr Ser Arg Asn Trp Gly Ser Ser
                 145                 150                 155

TTT AGC CAG GAC AGT GAC AGT GAG GGA CAT TCA GAA GAC TCT GAG AGG               528
Phe Ser Gln Asp Ser Asp Ser Glu Gly His Ser Glu Asp Ser Glu Arg
                 160                 165                 170

TGG TCT GGG TCT GCT TCC AGA AAC CAT CAT GGA TCT GCT CAG GAG CAG               576
Trp Ser Gly Ser Ala Ser Arg Asn His His Gly Ser Ala Gln Glu Gln
         175                 180                 185

CTA AGA GAT GGC TCC AGA CAC CCC AGG TCC CAT CAA GAA GAC AGA GCT               624
Leu Arg Asp Gly Ser Arg His Pro Arg Ser His Gln Glu Asp Arg Ala
         190                 195                 200

GGT CAT GGG CAC TCT GCA GAC AGC TCC AGA CAA TCA GGC ACT CGT CAC               672
Gly His Gly His Ser Ala Asp Ser Ser Arg Gln Ser Gly Thr Arg His
205                 210                 215                 220

ACA CAG ACT TCC TCT GGT GGA CAG GCT GCA TCA TCC CAT GAA CAG GCA               720
```

```
    Thr Gln Thr Ser Ser Gly Gly Gln Ala Ala Ser Ser His Glu Gln Ala
                225                 230                 235

AGA TCA AGT GCA GGA GAA AGA CAT GGA TCC CAC CAC CAG CAG TCA GCA        768
Arg Ser Ser Ala Gly Glu Arg His Gly Ser His His Gln Gln Ser Ala
            240                 245                 250

GAC AGC TCC AGA CAC TCA GGC ATT GGG CAC GGA CAA GCT TCA TCT GCA        816
Asp Ser Ser Arg His Ser Gly Ile Gly His Gly Gln Ala Ser Ser Ala
            255                 260                 265

GTC AGA GAC AGT GGA CAC CGA GGG TAC AGT GGT AGT CAG GCC AGT GAC        864
Val Arg Asp Ser Gly His Arg Gly Tyr Ser Gly Ser Gln Ala Ser Asp
            270                 275                 280

AAT GAG GGA CAT TCA GAA GAC TCA GAC ACA CAG TCA GTG TCA GCC CAC        912
Asn Glu Gly His Ser Glu Asp Ser Asp Thr Gln Ser Val Ser Ala His
285                 290                 295                 300

GGA CAG GCT GGG TCC CAT CAG CAG AGC CAC CAA GAG TCC GCA CGT GGC        960
Gly Gln Ala Gly Ser His Gln Gln Ser His Gln Glu Ser Ala Arg Gly
                305                 310                 315

CGG TCA GGG GAA ACG TCT GGA CAT TCA GGA TCT CCC GGG CTC TAG  A        1006
Arg Ser Gly Glu Thr Ser Gly His Ser Gly Ser Pro Gly Leu  *
                320                 325                 330
```

(2) INFORMATION FOR SEQ ID NO: 47:

      (i) SEQUENCE CHARACTERISTICS:
          (A) LENGTH: 331 amino acids
          (B) TYPE: amino acid
          (D) TOPOLOGY: linear

      (ii) MOLECULE TYPE: protein
      (xi) SEQUENCE DESCRIPTION: SEQ ID NO: 47:

```
Met Gly Ser Phe Leu Tyr Gln Val Gly Thr His Glu Gln Ser Glu Ser
 1               5                   10                  15

Ala His Gly Arg Thr Gly Thr Ser Thr Gly Gly Arg Gln Gly Ser His
            20                  25                  30

His Glu Gln Ala Arg Asp Ser Ser Arg His Ser Thr Ser Gln Glu Gly
        35                  40                  45

Gln Asp Thr Ile His Gly His Arg Gly Ser Ser Ser Gly Gly Arg Gln
        50                  55                  60

Gly Ser His Tyr Glu Gln Ser Val Asp Arg Ser Gly His Ser Gly Ser
 65                 70                  75                  80

His His Ser His Thr Thr Ser Gln Gly Arg Ser Asp Ala Ser His Gly
                85                  90                  95

Gln Ser Gly Ser Arg Ser Ala Ser Arg Gln Thr Arg Asn Asp Glu Gln
            100                 105                 110
```
```

```
Ser Gly Asp Gly Ser Arg His Ser Gly Ser Arg His His Glu Ala Ser
        115                 120                 125

Ser Arg Ala Asp Ser Ser Arg His Ser Gln Ala Val Gln Gly Gln Ser
        130                 135                 140

Glu Gly Pro Arg Thr Ser Arg Asn Trp Gly Ser Ser Phe Ser Gln Asp
145                 150                 155                 160

Ser Asp Ser Glu Gly His Ser Glu Asp Ser Glu Arg Trp Ser Gly Ser
                165                 170                 175

Ala Ser Arg Asn His His Gly Ser Ala Gln Glu Gln Leu Arg Asp Gly
            180                 185                 190

Ser Arg His Pro Arg Ser His Gln Glu Asp Arg Ala Gly His Gly His
            195                 200                 205

Ser Ala Asp Ser Ser Arg Gln Ser Gly Thr Arg His Thr Gln Thr Ser
        210                 215                 220

Ser Gly Gly Gln Ala Ala Ser Ser His Glu Gln Ala Arg Ser Ser Ala
225                 230                 235                 240

Gly Glu Arg His Gly Ser His His Gln Gln Ser Ala Asp Ser Ser Arg
                245                 250                 255

His Ser Gly Ile Gly His Gly Gln Ala Ser Ser Ala Val Arg Asp Ser
            260                 265                 270

Gly His Arg Gly Tyr Ser Gly Ser Gln Ala Ser Asp Asn Glu Gly His
            275                 280                 285

Ser Glu Asp Ser Asp Thr Gln Ser Val Ser Ala His Gly Gln Ala Gly
        290                 295                 300

Ser His Gln Gln Ser His Gln Glu Ser Ala Arg Gly Arg Ser Gly Glu
305                 310                 315                 320

Thr Ser Gly His Ser Gly Ser Pro Gly Leu  *
                325                 330
```

(2) INFORMATION FOR SEQ ID NO: 48:

        (i) SEQUENCE CHARACTERISTICS:
            (A) LENGTH: 1006 base pairs
            (B) TYPE: nucleic acid
            (C) STRANDEDNESS: single
            (D) TOPOLOGY: linear

        (ii) MOLECULE TYPE: DNA (genomic)

        (iii) HYPOTHETICAL: NO

        (iv) ANTI-SENSE: NO

        (ix) FEATURE:
            (A) NAME/KEY: CDS

48

(B) LOCATION:13..1006

(ix) FEATURE:
  (A) NAME/KEY: mat_peptide
  (B) LOCATION:13..1003


(xi) SEQUENCE DESCRIPTION: SEQ ID NO: 48:

```
GAATTCGCCA CC ATG GGG TCT TTC CTC TAC CAG GTG AGC ACT CAT GAA         48
              Met Gly Ser Phe Leu Tyr Gln Val Ser Thr His Glu
               1               5                   10


CAG TCT GAG TCC TCC CAT GGA TGG ACG GGG CCC AGC ACT AGA GGA AGA       96
Gln Ser Glu Ser Ser His Gly Trp Thr Gly Pro Ser Thr Arg Gly Arg
         15                  20                  25


CAA GGA TCC CGC CAT GAG CAG GCA CAA GAC AGC TCC AGG CAC TCA GCA      144
Gln Gly Ser Arg His Glu Gln Ala Gln Asp Ser Ser Arg His Ser Ala
     30                  35                  40


TCC CAA GAC GGT CAG GAC ACC ATT CGT GGA CAC CCG GGG TCA AGC AGA      192
Ser Gln Asp Gly Gln Asp Thr Ile Arg Gly His Pro Gly Ser Ser Arg
 45                  50                  55                  60


GGA GGA AGG CAG GGA TCC CAC TAC GAG CAA TCG GTA GAT AGA TCT GGA      240
Gly Gly Arg Gln Gly Ser His Tyr Glu Gln Ser Val Asp Arg Ser Gly
             65                  70                  75


CAC TCA GGG TCT CAT CAC AGC CAC ACC ACA TCC CAG GGA AGG TCT GAT      288
His Ser Gly Ser His His Ser His Thr Thr Ser Gln Gly Arg Ser Asp
             80                  85                  90


GCC TCC CAT GGG CAC TCA GGA TCC AGA AGT GCA AGC AGA CAA ACT CGT      336
Ala Ser His Gly His Ser Gly Ser Arg Ser Ala Ser Arg Gln Thr Arg
             95                  100                 105


AAC GAT GAA CAA TCA GGA GAC GGC TCC AGG CAC TCA GGG TCG CGT CAC      384
Asn Asp Glu Gln Ser Gly Asp Gly Ser Arg His Ser Gly Ser Arg His
     110                 115                 120


CAT GAA GCT TCC TCT CGG GCC GAC AGC TCT AGA CAC TCG CAG GTG GGC      432
His Glu Ala Ser Ser Arg Ala Asp Ser Ser Arg His Ser Gln Val Gly
125                 130                 135                 140


CAG GAA CAA TCA GAG GGG CCC AGG ACA AGC AGG AAC TGG GGA TCC AGT      480
Gln Glu Gln Ser Glu Gly Pro Arg Thr Ser Arg Asn Trp Gly Ser Ser
             145                 150                 155


TTT AGC CAG GAC AGT GAC AGT CAG GGA CAC TCA GAA GAC TCT GAG AGG      528
Phe Ser Gln Asp Ser Asp Ser Gln Gly His Ser Glu Asp Ser Glu Arg
             160                 165                 170


TGG TCT GGG TCT GCT TCC AGA AAC CAT CAT GGA TCT GCT CAG GAG CAG      576
Trp Ser Gly Ser Ala Ser Arg Asn His His Gly Ser Ala Gln Glu Gln
             175                 180                 185


CTA AGA GAT GGC TCC AGA CAC CCC AGG TCC CAT CAA GAA GAC AGA GCT      624
Leu Arg Asp Gly Ser Arg His Pro Arg Ser His Gln Glu Asp Arg Ala
```

```
              190                195                200

GGT CAT GGG CAC TCT GCA GAC AGC TCC AGA CAA TCA GGC ACT CGT CAC      672
Gly His Gly His Ser Ala Asp Ser Ser Arg Gln Ser Gly Thr Arg His
205                210                215                220

ACA CAG ACT TCC TCT GGT GGA CAG GCT GCA TCA TCC CAT GAA CAG GCA      720
Thr Gln Thr Ser Ser Gly Gly Gln Ala Ala Ser Ser His Glu Gln Ala
               225                230                235

AGA TCA AGT GCA GGA GAA AGA CAT GGA TCC CAC CAC CAG CAG TCA GCA      768
Arg Ser Ser Ala Gly Glu Arg His Gly Ser His His Gln Gln Ser Ala
           240                245                250

GAC AGC TCC AGA CAC TCA GGC ATT GGG CAC GGA CAA GCT TCA TCT GCA      816
Asp Ser Ser Arg His Ser Gly Ile Gly His Gly Gln Ala Ser Ser Ala
           255                260                265

GTC AGA GAC AGT GGA CAC CGA GGG TAC AGT GGT AGT CAG GCC AGT GAC      864
Val Arg Asp Ser Gly His Arg Gly Tyr Ser Gly Ser Gln Ala Ser Asp
       270                275                280

AAT GAG GGA CAT TCA GAA GAC TCA GAC ACA CAG TCA GTG TCA GCC CAC      912
Asn Glu Gly His Ser Glu Asp Ser Asp Thr Gln Ser Val Ser Ala His
285                290                295                300

GGA CAG GCT GGG TCC CAT CAG CAG AGC CAC CAA GAG TCC GCA CGT GGC      960
Gly Gln Ala Gly Ser His Gln Gln Ser His Gln Glu Ser Ala Arg Gly
               305                310                315

CGG TCA GGG GAA ACG TCT GGA CAT TCA GGA TCT CCC GGG CTC TAG  A     1006
Arg Ser Gly Glu Thr Ser Gly His Ser Gly Ser Pro Gly Leu  *
               320                325                330
```

(2) INFORMATION FOR SEQ ID NO: 49:

    (i) SEQUENCE CHARACTERISTICS:
        (A) LENGTH: 331 amino acids
        (B) TYPE: amino acid
        (D) TOPOLOGY: linear

    (ii) MOLECULE TYPE: protein
    (xi) SEQUENCE DESCRIPTION: SEQ ID NO: 49:

```
Met Gly Ser Phe Leu Tyr Gln Val Ser Thr His Glu Gln Ser Glu Ser
 1               5                10                15

Ser His Gly Trp Thr Gly Pro Ser Thr Arg Gly Arg Gln Gly Ser Arg
               20                25                30

His Glu Gln Ala Gln Asp Ser Ser Arg His Ser Ala Ser Gln Asp Gly
           35                40                45

Gln Asp Thr Ile Arg Gly His Pro Gly Ser Ser Arg Gly Gly Arg Gln
       50                55                60

Gly Ser His Tyr Glu Gln Ser Val Asp Arg Ser Gly His Ser Gly Ser
 65               70                75                80
```

```
His His Ser His Thr Thr Ser Gln Gly Arg Ser Asp Ala Ser His Gly
                85                  90                  95

His Ser Gly Ser Arg Ser Ala Ser Arg Gln Thr Arg Asn Asp Glu Gln
            100                 105                 110

Ser Gly Asp Gly Ser Arg His Ser Gly Ser Arg His His Glu Ala Ser
            115                 120                 125

Ser Arg Ala Asp Ser Ser Arg His Ser Gln Val Gly Gln Glu Gln Ser
        130                 135                 140

Glu Gly Pro Arg Thr Ser Arg Asn Trp Gly Ser Ser Phe Ser Gln Asp
145                 150                 155                 160

Ser Asp Ser Gln Gly His Ser Glu Asp Ser Glu Arg Trp Ser Gly Ser
            165                 170                 175

Ala Ser Arg Asn His His Gly Ser Ala Gln Glu Gln Leu Arg Asp Gly
            180                 185                 190

Ser Arg His Pro Arg Ser His Gln Glu Asp Arg Ala Gly His Gly His
        195                 200                 205

Ser Ala Asp Ser Ser Arg Gln Ser Gly Thr Arg His Thr Gln Thr Ser
        210                 215                 220

Ser Gly Gly Gln Ala Ala Ser Ser His Glu Gln Ala Arg Ser Ser Ala
225             230                 235                 240

Gly Glu Arg His Gly Ser His His Gln Gln Ser Ala Asp Ser Ser Arg
            245                 250                 255

His Ser Gly Ile Gly His Gly Gln Ala Ser Ser Ala Val Arg Asp Ser
            260                 265                 270

Gly His Arg Gly Tyr Ser Gly Ser Gln Ala Ser Asp Asn Glu Gly His
        275                 280                 285

Ser Glu Asp Ser Asp Thr Gln Ser Val Ser Ala His Gly Gln Ala Gly
        290                 295                 300

Ser His Gln Gln Ser His Gln Glu Ser Ala Arg Gly Arg Ser Gly Glu
305                 310                 315                 320

Thr Ser Gly His Ser Gly Ser Pro Gly Leu  *
            325                 330
```

## Claims

1. Peptide comprising a sequence of less than 50 amino acids of any variant of natural filaggrin, comprising at least one citrulline residue, and wherein the presence of said citrulline is crucial for reacting with antibodies that are present in sera from patients with rheumatoid arthritis.

2. Peptide according to claim 1 comprising the amino acid sequence
HSASQDGQDTIXGHPGSS or,
HSGIGHGQASSAVRDSGHXGYS or,
DSGHXGYSGSQASDNEGH or,
HSTSQEGQDTIHGHXGS or,
GGQGSXHQQAR or,
QGSXHQQARDSSRHSTSQEGQDTIHGHXGS or,
QGSXHQQARDSSRHSASQDGQDTIXGHPGSS or,
HSGIGHGQASSAVRDSGHXGYSGSQASDNEGH or,
an analog of said peptides comprising amino acid substitutions, that are characteristic for allelic variants of filaggrin,
wherein X represents a citrulline residue.

3. Peptide and/or chemical structure comprising any of the peptides according to claims 1 to 2, fused to a linker molecule.

4. Circularized peptide that comprises at least one of the peptides according to any of the claims 1 to 3.

5. Peptide comprising and/or consisting of tandem repeats of at least two of any of the peptides of claims 1 to 4.

6. Branched peptide that comprises at least one of the peptides according to any of the claims 1 to 5.

7. Method for producing a peptide according to any of claims 1 to 6, by classical chemical synthesis, wherein citrulline residues are substituted for arginine residues at certain steps during the chemical synthesis.

8. Method for producing a peptide according to any of claims 1 to 6, wherein the primary amino acid sequence is produced by classical chemical synthesis, and wherein arginine residues are subsequently derivatized towards citrulline residues by contacting said peptide with a peptidylarginine deiminase.

9. Method for producing a peptide of any of claims 1 to 6 comprising the following steps:

   - transforming an appropriate cellular host with a recombinant vector in which a polynucleic acid is inserted comprising the sequence that codes for said peptide under the control of the appropriate regulatory elements such that said peptide or a protein comprising said peptide is expressed and/or secreted,
   - culturing said transformed cellular host under conditions allowing expression of said protein or peptide and allowing derivatization of arginine residues towards citrulline residues,
   - harvesting said peptide.

10. Method for producing a peptide of any of claims 1 to 6 comprising the following steps:

   - transforming an appropriate cellular host with a recombinant vector in which a polynucleic acid is inserted comprising the sequence that codes for said peptide under the control of the appropriate regulatory elements, such that said peptide or a protein comprising said peptide is expressed and/or secreted,
   - culturing said transformed cellular host under conditions allowing expression of said protein or said peptide,
   - harvesting said protein or said peptide,
   - derivatizing arginine residues of said protein or said peptide by contacting with a peptidylarginine deiminase.

11. Method according to any of claims 9 or 10 wherein said host cell is a bacterial host or yeast or any other eukaryotic host cell which is preferably transformed with a recombinant baculovirus.

12. An antibody raised upon immunization with a peptide according to any of the claims 1 to 6, with said antibody being specifically reactive with the peptide forms that contain citrulline residues, and with said antibody being preferably a monoclonal antibody.

13. Anti-idiotype antibody raised upon immunization with an antibody according to claim 12, with said anti-idiotype antibody being specifically reactive with the antibody of claim 12, thereby mimicking the peptide that contains citrulline according to any of claims 1 to 6, and with said antibody being preferably a monoclonal antibody.

14. An immunotoxin molecule comprising and/or consisting of cell recognition molecule being a peptide of any of claims 1 to 6, or an antibody according to any of the claims 12 or 13, covalently bound to a toxin molecule or active fragment thereof.

15. A peptide according to any of the claims 1 to 6 or an antibody according to any of claims 12 or 13 or an immunotoxin molecule according to claim 14 or a composition thereof for use as a medicament.

16. Use of a peptide according to any of claims 1 to 6 or an antibody according to any of claims 12 or 13 or an immunotoxin molecule according to claim 14 or a composition thereof for the preparation of a medicament or of a diagnosticum for rheumatoid arthritis.

17. Use of a polypeptide according to any of the claims 4 to 6 or a composition thereof for the preparation of a medicament to treat rheumatoid arthritis by increasing the size of antigen-immune complexes, thereby improving the clearance of the formed immune complexes.

18. A diagnostic kit for use in detecting auto-immune diseases such as:

- rheumatoid arthritis,
- systemic lupus erythematosus,
- discoid lupus erythematosus,
- scleroderma,
- dermatomyositis,
- Sjögren's syndrome,

said kit comprising at least one peptide according to any of claims 1 to 6, or an antibody according to any of claims 12 or 14, with said peptide or antibody being possibly bound to a solid support.

19. A diagnostic kit according to claim 18, said kit comprising a range of peptides according to any of claims 1 to 6 or of antibodies according to any of claims 12 to 14, possibly in combination with antigens that constitute immunogenic determinants for other auto-immune diseases, wherein said peptides are attached to specific locations on a solid substrate.

20. A diagnostic kit according to claim 18 or 19, wherein said solid support is a membrane strip and said polypeptides are coupled to the membrane in the form of parallel lines.

21. A diagnostic kit according to claims 18 or 19 wherein certain peptides are not attached to a solid support but are provided in the binding solution to be used as competitors and/or to block other antibodies that are present in sera from patients with autoimmune disease other than rheumatoiud arthritis, thereby decreasing or eliminating possible cross-reaction and/or aspecific binding.

Figure 1a

EP 0 949 270 A1

Figure 1b

```
HB2641     MG--------------------SFLYQVSTHEQSESAHGRTGTSTGGR    28

HB2642     MG--------------------SFLYQVSTHEQSESAHGRTGTSTGGR    28

HB2650     MG--------------------SFLYQVGTHEQSESAHGRTGTSTGGR    28

HFIL1      AGPHQQSHQESTRGRSAGRSGRSGSFLYQVSTHEQSESAHGRTGTSTGGR    50

HB2648     MG--------------------SFLYQVSTHEQSESSHGWTGPSTRGR    28

FILGAN1    ------------------------FLYQVSTHEQSESSHGRSGTSTGGR    25

HPROFIL22  --------------------GSFLYQVSTHEQSESAHGRSAPSTRRR    27

HPROFIL21  --------------------GSFLYQVSTHEQSESSHGWARTSTGRR    27

FILGAN2    ---------------------FLIQVSTHEQSDSAHGQARPSTRRR    25

FILGAN3    ---------------------FLIQVSTHEQSDSTHGWTAPSTRRR    25

FILGAN4    ---------------------FLIQVSTHEQSDSTHGWTVPSTRRR    25

                             ** **.*****.*.**  . .**   *


HB2641     QGSHHEQARDSSRHSTSQEGQDTIHGHRGSSSGGRQGSHYEQSVDRSGHS    78

HB2642     QGSHHEQARDSSRHSTSQEGQDTIHGHRGSSSGGRQGSHYEQSVDRSGHS    78

HB2650     QGSHHEQARDSSRHSTSQEGQDTIHGHRGSSSGGRQGSHYEQSVDRSGHS    78
                   5         4
HFIL1      ██████DSSR███████████████SSGGRQGSHYEQLVDRSGHS        100
                █                1
HB2648     QGS█HEQAQDSSR██████████████SRGGRQGSHYEQSVDRSGHS      78

FILGAN1    QGSHHEQARDSSRHSTSQEGQDTIHGHPGSSSGGRQGSHYEQSVDRSGHS    75

HPROFIL22  QGSHHDQARDSSRHSASQEGQDTIRGHPGSSRGGRQGSHYEQSVDRSGHS    77

HPROFIL21  QGSRHDQAQDSSRHSTSQQGQDTIHAHPGSRRGGRHGYHHEQSADSTGHS    77

FILGAN2    QGSRHDQAQDSSGHSASQQGQDTIRARPGPRRRGRHGYYHEQLANSTGHR    75

FILGAN3    QGSRHQQAQDSSGHSASQDGQDTIRARPGPRRRGRHGYYHEQLANSTGHR    75

FILGAN4    QGSRHQQAQDSSGHSASQDGQDTIRARPGPRRRGRHGYYHEQLANSTGHR    75

           ***.*.**.*** **.**.*****....*... **.* ..** ....**.
```

Figure 2

Figure 3

INHIBITION ELISA
WITH NATIVE FILAGGRIN

OD (450 nm)

no inhibition
filaggrin inhib. 1µg/ml
filaggrin inhib. 10µg/ml

APF  35036  35037  35038  35044  35055

Figure 4

INHIBITION ELISA
WITH FILAGGRIN PEPTIDES

Figure 5

EP 0 949 270 A1

Figure 6

```
HB2641    GAATTCGCCACCATGGGGTCTTTCCTCTACCAGGTGAGCACTCATGAACA     50
HB2642    GAATTCGCCACCATGGGGGTCTTTCCTCTACCAGGTGAGCACTCATGAACA    50
HB2650    GAATTCGCCACCATGGGGGTCTTTCCTCTACCAGGTGGGCACTCATGAACA    50
HB2648    GAATTCGCCACCATGGGGGTCTTTCCTCTACCAGGTGAGCACTCATGAACA    50
HFIL1     --------------GGGTCTTTCCTCTACCAGGTGAGCACTCATGAACA       35
                        **********************.*************


HB2641    GTCTGAGTCCGCCCATGGACGGACCGGGACCAGCACTGGAGGAAGACAAG    100
HB2642    GTCTGAGTCCGCCCATGGACGGACCGGGACCAGCACTGGAGGAAGACAAG    100
HB2650    GTCTGAGTCCGCCCATGGACGGACCGGGACCAGCACTGGAGGAAGACAAG    100
HB2648    GTCTGAGTCCTCCCATGGATGGACGGGGCCCAGCACTAGAGGAAGACAAG    100
HFIL1     GTCTGAGTCCGCCCATGGACGGACCGGGACCAGCACTGGAGGAAGACAGG    85
          *********.******* **** *** ****.*************.*


HB2641    GATCCCACCACGAGCAGGCACGAGACAGCTCCAGGCACTCAACGTCCCAA    150
HB2642    GATCCCACCACGAGCAGGCACGAGACAGCTCCAGGCACTCAACGTCCCAA    150
HB2650    GATCCCACCACGAGCAGGCACGAGACAGCTCCAGGCACTCAACGTCCCAA    150
HB2648    GATCCCGCCATGAGCAGGCACAAGACAGCTCCAGGCACTCAGCATCCCAA    150
HFIL1     GATCCCACCACCAGCAGGCACGAGACAGCTCCAGGCACTCAACGTCCCAA    135
          ******.***   ********.******************.*.******


HB2641    GAGGGTCAGGACACCATTCATGGACACCGGGGGGTCAAGCAGTGGAGGAAG    200
HB2642    GAGGGTCAGGACACCATTCATGGACACCGGGGGGTCAAGCAGTGGAGGAAG    200
HB2650    GAGGGTCAGGACACCATTCATGGACACCGGGGGGTCAAGCAGTGGAGGAAG    200
HB2648    GACGGTCAGGACACCATTCGTGGACACCCGGGGGTCAAGCAGAGGAGGAAG    200
HFIL1     GAGGGTCAGGACACCATTCATGGACACCGGGGGGTCAAGCAGTGGAGGAAG    185
          **  ***************.*******  ***********.*.*******
```

Figure 6 cont'd

```
HB2641   GCAGGGATCCCACTACGAGCAATCGGTAGATAGATCTGGACACTCAGGGT    250
HB2642   GCAGGGATCCCACTACGAGCAATCGGTAGATAGATCTGGACACTCAGGGT    250
HB2650   GCAGGGATCCCACTACGAGCAATCGGTAGATAGATCTGGACACTCAGGGT    250
HB2648   GCAGGGATCCCACTACGAGCAATCGGTAGATAGATCTGGACACTCAGGGT    250
HFIL1    GCAGGGATCCCACTACGAGCAATTGGTAGATAGATCTGGACACTCAGGGT    235

         ******************** *************************
```

```
HB2641   CTCATCACAGCCACACCACATCCCAGGGAAGGTCTGATGCCTCCCATGGG    300
HB2642   CTCATCACAGCCACACCACATCCCAGGGAAGGTCTGATGCCTCCCATGGG    300
HB2650   CTCATCACAGCCACACCACATCCCAGGGAAGGTCTGATGCCTCCCATGGG    300
HB2648   CTCATCACAGCCACACCACATCCCAGGGAAGGTCTGATGCCTCCCATGGG    300
HFIL1    CTCATCACAGCCACACGACCTCACAGGGAAGGTCTGATGCCTCCCATGGG    285

         *************** ** ** *************************
```

```
HB2641   CAGTCAGGATCCAGAAGTGCAAGCAGACAAACTCGTAACGATGAACAATC    350
HB2642   CAGTCAGGATCCAGAAGTGCAAGCAGACAAACTCGTAACGATGAACAATC    350
HB2650   CAGTCAGGATCCAGAAGTGCAAGCAGACAAACTCGTAACGATGAACAATC    350
HB2648   CACTCAGGATCCAGAAGTGCAAGCAGACAAACTCGTAACGATGAACAATC    350
HFIL1    CACTCAGGATCCAGAAGTGCAAGCAGACAAACTCGTAACGATGAACAATC    335

         ** ***********************************************
```

```
HB2641   AGGAGACGGCTCCAGGCACTCAGGGTCGCGTCACCATGAAGCTTCCTCTC    400
HB2642   AGGAGACGGCTCCAGGCACTCAGGGTCGCGCCACCATGAAGCTTCCTCTC    400
HB2650   AGGAGACGGCTCCAGGCACTCAGGGTCGCGTCACCATGAAGCTTCCTCTC    400
HB2648   AGGAGACGGCTCCAGGCACTCAGGGTCGCGTCACCATGAAGCTTCCTCTC    400
HFIL1    AGGAGACGGCTCCAGGCACTCAGGGTCGCGTCACCATGAAGCTTCCTCTC    385

         ****************************** ******************
```

62

Figure 6 cont'd

```
HB2641   GGGCCGACAGCTCTAGACACTCGCAGGTGGGCCAGGGACAATCAGAGGGG         450
HB2642   GGGCCGACAGCTCTAGACACTCGCAGGTGGGCCAGGGACAATCAGAGGGG         450
HB2650   GGGCCGACAGCTCTAGACACTCACAGGCAGTCCAGGGACAATCAGAGGGG         450
HB2648   GGGCCGACAGCTCTAGACACTCGCAGGTGGGCCAGGAACAATCAGAGGGG         450
HFIL1    GGGCCGACAGCTCTGGACACTCGCAGGTGGGCCAGGGACAATCAGAGGGG         435
         ************* ******* ****  * ***** *************

HB2641   CCCAGGACAAGCAGGAACTGGGGATCCAGTTTTAGCCAGGACAGTGACAG         500
HB2642   CCCAGGACAAGCAGGAACTGGGGATCCAGTTTTAGCCAGGACAGTGACAG         500
HB2650   CCCAGGACAAGCAGGAACTGGGGATCCAGTTTTAGCCAGGACAGTGACAG         500
HB2648   CCCAGGACAAGCAGGAACTGGGGATCCAGTTTTAGCCAGGACAGTGACAG         500
HFIL1    CCCAGGACAAGCAGGAACTGGGGATCCAGTTTTAGCCAGGACAGTGACAG         485
         **************************************************

HB2641   TGAGGGACATTCAGAAGACTCTGAGAGGTGGTCTGGGTCTGCTTCCAGAA         550
HB2642   TGAGGGACATTCAGAAGACTCTGAGAGGTGGTCTGGGTCTGCTTCCAGAA         550
HB2650   TGAGGGACATTCAGAAGACTCTGAGAGGTGGTCTGGGTCTGCTTCCAGAA         550
HB2648   TCAGGGACACTCAGAAGACTCTGAGAGGTGGTCTGGGTCTGCTTCCAGAA         550
HFIL1    TCAGGGACACTCAGAAGACTCTGAGAGGTGGTCTGGGTCTGCTTCCAGAA         535
         *  ******* ***************************************

HB2641   ACCATCATGGATCTGCTCAGGAGCAGCTAAGAGATGGCTCCAGACACCCC         600
HB2642   ACCATCATGGATCTGCTCAGGAGCAGCTAAGAGATGGCTCCAGACACCCC         600
HB2650   ACCATCATGGATCTGCTCAGGAGCAGCTAAGAGATGGCTCCAGACACCCC         600
HB2648   ACCATCATGGATCTGCTCAGGAGCAGCTAAGAGATGGCTCCAGACACCCC         600
HFIL1    ACCATCATGGATCTGCTCAGGAGCAGCTAAGAGATGGCTCCAGACACCCC         585
         **************************************************
```

63

Figure 6 cont'd

```
HB2641    AGGTCCCATCAAGAAGACAGAGCTGGTCATGGGCACTCTGCAGACAGCTC    650
HB2642    AGGTCCCATCAAGAAGGCAGAGCTGGTCATGGGCACTCTGCAGACAGCTC    650
HB2650    AGGTCCCATCAAGAAGACAGAGCTGGTCATGGGCACTCTGCAGACAGCTC    650
HB2648    AGGTCCCATCAAGAAGACAGAGCTGGTCATGGGCACTCTGCAGACAGCTC    650
HFIL1     AGGTCCCATCAAGAAGACAGAGCTGGTCATGGGCACTCTGCAGACAGCTC    635
          **************** *********************************


HB2641    CAGACAATCAGGCACTCGTCACACACAGACTTCCTCTGGTGGACAGGCTG    700
HB2642    CAGACAATCAGGCACTCGTCACACACAGACTTCCTCTGGTGGACAGGCTG    700
HB2650    CAGACAATCAGGCACTCGTCACACACAGACTTCCTCTGGTGGACAGGCTG    700
HB2648    CAGACAATCAGGCACTCGTCACACACAGACTTCCTCTGGTGGACAGGCTG    700
HFIL1     CAGACAATCAGGCACTCGTCACACACAGACTTCCTCTGGTGGACAGGCTG    685
          **************************************************


HB2641    CATCATCCCATGAACAGGCAAGATCAAGTGCAGGAGAAAGACATGGATCC    750
HB2642    CATCATCCCATGAACGGGCAAGATCAAGTGCAGGAGAAAGACATGGATCC    750
HB2650    CATCATCCCATGAACAGGCAAGATCAAGTGCAGGAGAAAGACATGGATCC    750
HB2648    CATCATCCCATGAACAGGCAAGATCAAGTGCAGGAGAAAGACATGGATCC    750
HFIL1     CATCATCCCATGAACAGGCAAGATCAAGTGCAGGAGACAGACATGGATCC    735
          **************** ********************** ************


HB2641    CACCACCAGCAGTCAGCAGACAGCTCCAGACACTCAGGCATTGGGCACGG    800
HB2642    CACCACCAGCAGTCAGCAGACAGCTCCAGACACTCAGGCATTGGGCACGG    800
HB2650    CACCACCAGCAGTCAGCAGACAGCTCCAGACACTCAGGCATTGGGCACGG    800
HB2648    CACCACCAGCAGTCAGCAGACAGCTCCAGACACTCAGGCATTGGGCACGG    800
HFIL1     CACCACCAGCAGTCAGCAGACAGCTCCAGACACTCAGGCATTGGGCACGG    785
          **************************************************
```

EP 0 949 270 A1

Figure 6 cont'd

```
HB2641   ACAAGCTTCATCTGCAGTCAGAGACAGTGGACACCGAGGGTACAGTGGTA   850
HB2642   ACAAGCTTCATCTGCAGTCAGAGACAGTGGACACCGAGGGTACAGTGGTA   850
HB2650   ACAAGCTTCATCTGCAGTCAGAGACAGTGGACACCGAGGGTACAGTGGTA   850
HB2648   ACAAGCTTCATCTGCAGTCAGAGACAGTGGACACCGAGGGTACAGTGGTA   850
HFIL1    ACAAGCTTCATCTGCAGTCAGAGACAGTGGACACCGAGGGTACAGTGGTA   835
         **************************************************


HB2641   GTCAGGCCAGTGACAATGAGGGACATTCAGAAGACTCAGACACACAGTCA   900
HB2642   GTCAGGCCAGTGACAATGAGGGACATTCAGAAGACTCAGACACACAGTCA   900
HB2650   GTCAGGCCAGTGACAATGAGGGACATTCAGAAGACTCAGACACACAGTCA   900
HB2648   GTCAGGCCAGTGACAATGAGGGACATTCAGAAGACTCAGACACACAGTCA   900
HFIL1    GTCAGGCCAGTGACAATGAGGGACATTCAGAAGACTCAGACACACAGTCA   885
         **************************************************


HB2641   GTGTCAGCCCACGGACAGGCTGGGTCCCATCAGCAGTGCCACCAAGAGTC   950
HB2642   GTGTCAGCCCACGGACAGGCTGGGTCCCATCAGCAGAGCCACCAAGAGTC   950
HB2650   GTGTCAGCCCACGGACAGGCTGGGTCCCATCAGCAGAGCCACCAAGAGTC   950
HB2648   GTGTCAGCCCACGGACAGGCTGGGTCCCATCAGCAGAGCCACCAAGAGTC   950
HFIL1    GTGTCAGCCCACGGACAGGCTGGGTCCCATCAGCAGAGCCACCAAGAGTC   935
         ****************************************.*************


HB2641   CGCACGTGGCCGGTCAGGGGAAACGTCTGGACATTCAGGATCT--CC---   994
HB2642   CGCACGTGGCCGGTCAGGGGAAACGTCTGGACATTCAGGATCT--CC---   994
HB2650   CGCACGTGGCCGGTCAGGGGAAACGTCTGGACATTCAGGATCT--CC---   994
HB2648   CGCACGTGGCCGGTCAGGGGAAACGTCTGGACATTCAGGATCT--CC---   994
HFIL1    CGCACGTGGCCGGTCAGGGGAAACGTCTGGACATTCAGGATCTTTCCTCT   985
         ******************************************** **
```

```
HB2641      --CGGG----CTCTAGA      1006
HB2642      --CGGG----CTCTAGA      1006
HB2650      --CGGG----CTCTAGA      1006
HB2648      --CGGG----CTCTAGA      1006
HFIL1       ACCAGGTGAGCACT---       999
            *.**       *.**
```

Figure 6 control

**European Patent Office**

## EUROPEAN SEARCH REPORT

Application Number

EP 98 87 0078

### DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | SCHELLEKENS G A ET AL: "THE MODIFIED ARGININE RESIDUE CITRULLINES IS THE MAJOR CONSTITUENT OF EPITOPES RECOGNIZED BY AUTOANTIBODIES IN SERA FROM RHEUMATOID ARTHRITIS PATIENTS" ARTHRITIS AND RHEUMATISM, vol. 40, no. 9, SUPPL. 08, 8 November 1997, page S276 XP002067877 * abstract * | 1-10 | C07K14/47 C07K1/107 C07K16/18 A61K38/17 G01N33/564 |
| X | WO 98 08946 A (JOLIVET REYNAUD COLETTE ;VINCENT CHRISTIAN (FR); ARNAUD MICHEL (FR) 5 March 1998 * the whole document * * page 4, line 20 - line 30 * * page 6 - page 7; claims * | 1-10 | |
| X | WO 92 19649 A (CLONATEC SA) 12 November 1992 * page 4, line 22 - line 33 * * abstract * | 1-10 | |
| | | | TECHNICAL FIELDS SEARCHED (Int.Cl.6) C07K A61K G01N |

| Place of search | Date of completion of the search | Examiner |
|---|---|---|
| THE HAGUE | 8 December 1998 | Cervigni, S |

EPO FORM 1503 03.82 (P04C01)

European Patent
Office

Application Number

EP 98 87 0078

## CLAIMS INCURRING FEES

The present European patent application comprised at the time of filing more than ten claims.

☐ Only part of the claims have been paid within the prescribed time limit. The present European search report has been drawn up for the first ten claims and for those claims for which claims fees have been paid, namely claim(s):

☒ No claims fees have been paid within the prescribed time limit. The present European search report has been drawn up for the first ten claims.

## LACK OF UNITY OF INVENTION

The Search Division considers that the present European patent application does not comply with the requirements of unity of invention and relates to several inventions or groups of inventions, namely:

☐ All further search fees have been paid within the fixed time limit. The present European search report has been drawn up for all claims.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

**ANNEX TO THE EUROPEAN SEARCH REPORT
ON EUROPEAN PATENT APPLICATION NO.**

EP 98 87 0078

This annex lists the patent family members relating to the patent documents cited in the above-mentioned European search report.
The members are as contained in the European Patent Office EDP file on
The European Patent Office is in no way liable for these particulars which are merely given for the purpose of information.

08-12-1998

| Patent document cited in search report | | Publication date | Patent family member(s) | | Publication date |
|---|---|---|---|---|---|
| WO 9808946 | A | 05-03-1998 | FR | 2752842 A | 06-03-1998 |
| WO 9219649 | A | 12-11-1992 | FR | 2675805 A | 30-10-1992 |
| | | | FR | 2681600 A | 26-03-1993 |
| | | | CA | 2084876 A | 27-10-1992 |
| | | | EP | 0511116 A | 28-10-1992 |
| | | | GR | 93300038 T | 07-06-1993 |
| | | | JP | 6502187 T | 10-03-1994 |

EPO FORM P0459

For more details about this annex : see Official Journal of the European Patent Office, No. 12/82